(19) Europäisches Patentamt

European Patent Office

Office européen des brevets

(11) **EP 4 505 940 B1**

(12) **EUROPEAN PATENT SPECIFICATION**

(45) Date of publication and mention
of the grant of the patent:
**25.02.2026 Bulletin 2026/09**

(21) Application number: **23190750.2**

(22) Date of filing: **10.08.2023**

(51) International Patent Classification (IPC):
*A61B 5/00* (2006.01)     *A61B 5/145* (2006.01)
*A61B 5/1455* (2006.01)     *G01N 21/17* (2006.01)

(52) Cooperative Patent Classification (CPC):
**A61B 5/14532; A61B 5/0095; A61B 5/1451;**
**A61B 5/1455; A61B 5/6843; G01N 21/1702;**
A61B 5/681; A61B 5/6824; A61B 5/6826;
A61B 2562/0233

(54) **DEVICE AND METHOD FOR DETECTING AN ANALYTE IN TISSUE USING AN ADJUSTABLE IRRADIATION POSITION**

VORRICHTUNG UND VERFAHREN ZUM NACHWEIS EINES ANALYTEN IN GEWEBE MITTELS EINER EINSTELLBAREN BESTRAHLUNGSPOSITION

DISPOSITIF ET PROCÉDÉ DE DÉTECTION D'UN ANALYTE DANS UN TISSU À L'AIDE D'UNE POSITION D'IRRADIATION RÉGLABLE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(43) Date of publication of application:
**12.02.2025 Bulletin 2025/07**

(73) Proprietor: **DiaMonTech AG**
**10245 Berlin (DE)**

(72) Inventors:
• **KALUZA, Michael**
**10711 Berlin (DE)**

• **JANIK, Sergius**
**10115 Berlin (DE)**
• **LUBINSKI, Thorsten**
**10245 Berlin (DE)**

(74) Representative: **Lucke, Andreas**
**Boehmert & Boehmert**
**Anwaltspartnerschaft mbB**
**Pettenkoferstrasse 22**
**80336 München (DE)**

(56) References cited:
WO-A1-2023/002024    CN-U- 208 808 486
US-A1- 2003 010 898    US-A1- 2008 262 324
US-A1- 2023 181 064    US-A1- 2023 190 145

**Description**

FIELD OF THE INVENTION

The present invention generally relates to devices and methods for detecting an analyte in tissue of a human or animal subject. In particular, the present invention relates to devices and methods for non-invasive measurement of analytes in body fluids, such as glucose concentrations in human skin, in particular in the interstitial fluid of human skin.

BACKGROUND OF THE INVENTION

The present invention relates to a method of detecting an analyte in tissue of a human or animal subject. The method comprises a measurement procedure, in which a surface of the skin (skin surface) of the subject is brought in contact with a measurement body, which permits heat and/or pressure waves generated by absorption of excitation radiation in the tissue to be transferred to said measurement body. Excitation radiation is irradiated into the tissue to be absorbed by the analyte contained therein, wherein the intensity of said excitation radiation may be time-modulated, and wherein said excitation radiation may comprise radiation of different analyte-characteristic wavelengths that are irradiated one or both of simultaneously and sequentially.

Analyte-characteristic wavelengths as understood herein are wavelengths that allow for determining the presence of an analyte by wavelength-selective absorption, and hence form the basis of the analysis. As such, analyte-characteristic wavelengths may in particular include wavelengths corresponding to absorption maxima of the analyte. Further analyte-characteristic wavelengths are wavelengths corresponding to local minima between two absorption peaks. Namely, the difference between a local absorption minimum and an adjacent peak is a good measure of the concentration of analyte in the tissue. Herein, the term "local minimum" indicates that the absorptivity of the analyte at the given wavelength is smaller than at nearby wavelengths, but is still appreciable, otherwise they would not be characteristic for the analyte. In particular, in preferred embodiments, the absorptivity at these local minima serving as analyte-characteristic wavelengths is more than 5%, preferably more than 10%, more preferably more than 20%, most preferably more than 30% of the highest absorption peak associated with any of the analyte-characteristic wavelengths relied on in the analyte measurement. While wavelengths exactly corresponding with the absorption peaks or local absorption minima are usually the preferred choices for the analyte-characteristic wavelengths, wavelengths close to the maxima/minima or between maxima and minima may also be used. Accordingly, as understood herein, "analyte-characteristic wavelengths" are also wavelengths where the difference in absorption to that at the closest absorption peak or the closest local absorption minimum is less than 30%, preferably less than 20% of the difference in absorption between the closest absorption peak and closest local absorption minimum. Analyte-characteristic wavelengths may also include wavelengths where the absorption of other substances with which the analyte is mixed in the tissue, is particularly low.

Further, a physical response of the measurement body, or of a component included therein, to heat and/or pressure waves received from said tissue upon absorption of said excitation radiation is detected using a detection device which generates a response signal based on said detected physical response, said response signal being indicative of the degree of absorption of excitation radiation. In the following, the steps of irradiating excitation radiation into the tissue to be absorbed by the analyte contained therein, detecting said physical response of the measurement body and generating said response signal may also be referred to as "performing an analyte measurement".

The present invention is not limited to any specific physical response to heat and/or pressure waves received from said tissue upon absorption of the excitation radiation, nor to any specific way of detecting this physical response in a manner that allows for generating a response signal that is indicative of the degree of absorption of excitation radiation. Various physical responses and corresponding detection methods have been previously proposed for these types of analyte measurement procedure by the present applicant and are briefly summarized below, and each of them may be applied in the present invention.

For example, the detection device may comprise a light source for generating a detection light beam (detection beam) travelling through at least a portion of said measurement body or a component included in said measurement body, and said physical response of the measurement body to heat and/or pressure waves received from said tissue upon absorption of said excitation radiation may be a local change in the refractive index of said measurement body or said component. In this case, the detection device may be configured for detecting one or both of a change in the light path and a change in the phase of the detection light beam due to said change in refractive index of the material of the measurement body or the component included in the same.

For example, in various methods and devices described in detail in two earlier applications of the present applicant, published as WO 2015/193310 A1 and WO 2017/097824 A1, the measurement body is transparent for said detection light beam, and the detection light beam is directed to be totally or partially reflected at a surface of said measurement body that is in contact with said tissue. In this case, the detection device may comprise a photodetector, in particular a position sensitive photodetector which is capable of detecting a degree of deflection, in particular a deflection angle of said detection light beam due to said local change in refractive index. Accordingly, in this case, the physical response to the heat and/or pressure waves received by the measurement body is a local change in refractive index, and the response signal is the detected degree of deflection which is indeed found to be indicative of the degree of absorption of the excitation radiation.

In alternative variants suggested by the present applicant, as for example disclosed in international application WO 2020/094265 A1, said detection device may comprise an interferometric device allowing for assessing said change in phase of the detection beam and generating a response signal indicative of said change in phase. In this case, the physical response of the measurement body (or a component included therein) to heat and/or pressure waves received from said tissue upon absorption of said excitation radiation is again a local change in index of refraction, while the response signal is in this case an interferometric signal reflecting a change in the phase of the detection beam due to the local change in refractive index.

In yet alternative embodiments, the measurement body or a component in said measurement body may have electrical properties that change in response to a local change in temperature and/or a change in pressure associated therewith, and said detection device comprises electrodes for capturing electrical signals representing said electrical properties. Various possible setups are disclosed in WO 2019/110597 A2. For example, the measurement body may comprise sections having piezoelectric properties, and pressure changes associated with received heat and/or pressure lead to electrical signals that can be recorded with the electrodes. In this case, the change in pressure resembles the physical response of the measurement body or of a component included therein, to heat and/or pressure received from the tissue upon absorption of the excitation radiation, which is detected using the piezoelectric properties of the measurement body and the electrode, and which leads to electrical signals representing the aforementioned response signal that is indicative of the degree of absorption of excitation radiation. In yet further variants, a temperature change due to received heat can be directly measured using very sensitive temperature sensors.

In some examples, the measurement body or a component in said measurement body may comprise a volume in which acoustic waves such as sound waves can propagate, for example a cavity or void, a recess or cut-out and/or an acoustic resonator. The physical response of the measurement body or of said component in the measurement body may thus be a sound wave, e.g. a longitudinal pressure wave, in said volume that is excited by a heat and/or pressure wave received from the tissue upon absorption of the excitation radiation. The detection device may comprise a microphone that is arranged in or in contact with said volume. The microphone may act as a transducer to convert sound waves into electrical signals, wherein the electrical signals may represent the aforementioned response signal that is indicative of the degree of absorption of excitation radiation.

Note that in the following description, the physical response of the measurement body to heat received from the tissue as a result of thermal contact between the measurement body and the tissue is described in detail. However, it is to be understood that in various embodiments of the device and method of the invention, the tissue is in pressure transmitting contact with the measurement body, and the physical response of the measurement body is a response to pressure waves received from the tissue. Herein, the expression "pressure transmitting contact" shall include all relations that allow for the transfer of pressure waves from the tissue to the measurement body, and in particular, an acoustically coupled relation, where the coupling could be established by a gas, a liquid or a solid body. All detailed explanations given in connection with the thermal contact and physical response to heat received by the measurement body from the tissue shall be understood in combination with scenarios including pressure transmitting contact and physical response to pressure waves, where applicable, without explicit mention.

Note that the term "analyte measurement procedure" or "analyte measurement" indicates that this measurement procedure is based on response signals obtained with excitation radiation at analyte-characteristic wavelengths.

The method may further comprise an analyzing step, in which said analyzing is carried out based, at least in part, on said response signal. Since in this case, the response signal is indicative of the degree of absorption of excitation radiation comprising "analyte-characteristic wavelengths", the response signal is directly related to the concentration of the analyte in the tissue. Accordingly, the analyzing step is based at least in part on a measure of the concentration of the analyte in the tissue, and in some non-limiting applications, it may actually amount to determining this concentration.

For example, the above method has been employed by the applicant in devices for non-invasive measuring of a glucose level of a user. In this specific application, the "analyte" is formed by glucose, and the "tissue" is the skin of the user. It has been previously demonstrated that this method allows for very precise measurement of glucose concentration in the interstitial fluid within the skin of a person, which is found to be directly related with and hence representative of the glucose content of the patient's blood. Shown in Fig. 4 of the present application is the result of a Clark's error grid analysis taken from WO 2017/097824 A1, demonstrating that the above analysis method allows for predicting the actual glucose concentration of a person very precisely.

To excite the physical response in the measurement body, the contact between the measurement body and the skin surface has to permit heat and/or pressure waves generated by absorption of excitation radiation in the tissue to be transferred to said measurement body.

The surface of the skin of human (or animal) subjects, however, may exhibit a microstructure or micropattern, which may for example be formed by a plurality of raised and/or recessed portions (e.g. ridges and/or furrows) such as epidermal, papillary or friction ridges. A prominent example thereof is the fingerprint on the human finger, but similar microstructures may be present everywhere on the skin of human (or animal) subjects. In addition, the skin surface may also exhibit other irregularities, for example scars and nevi. The present inventors have found that such irregularities may affect analyte measurements, in particular the transmission of heat and/or pressure waves to the measurement body. For example, small air pockets may form between the measurement body and the skin surface (e.g. at recessed portions of the skin surface), which may significantly reduce the transmission efficiency locally and may thus affect the accuracy and reliability of the analyte measurement. This may in particular be an issue when implementing a method as described above in ever smaller devices (e.g. in handheld or wearable devices), for example since smaller radiation sources for generating excitation radiation may provide less optical power and thus generate smaller physical responses than larger radiation sources in standalone device.

10015a] US 2003/010898 A1 discloses a system for measuring a biological parameter, such as blood glucose, the system comprising the steps of directing laser pulses from a light guide into a body part consisting of soft tissue, such as the tip of a finger to produce a photoacoustic interaction. The resulting acoustic signal is detected by a transducer and analyzed to provide the desired parameter.

10015b] US 2023/181064 A1 discloses an apparatus for analyzing a material comprising at least one analyte, said apparatus comprising a measurement body having a contact surface suitable to be brought in thermal contact or pressure-transmitting contact with said material, an excitation radiation source configured for irradiating excitation radiation into the material to be absorbed therein, and a detection light source for generating a detection light beam travelling through at least a portion of said measurement body or a component included in said measurement body, wherein said detection light beam is directed to be totally or partially reflected at said contact surface, wherein said contact surface of the measurement body is curved in at least one principal direction in the area where the detection light beam is reflected.

10015c] WO 2023/002024 A1 discloses a device and a method for determining one or more parameters regarding a content and/or property of biomolecules, in particular metabolites, in a biological tissue. The device comprises at least one irradiation unit configured to irradiate the tissue with electromagnetic radiation exhibiting one or more wavelengths or wavelength ranges, in particular in the mid-infrared, near-infrared and/or visible spectral range, at least one detection unit configured to detect, in a spatially resolved manner, first signals corresponding to optical and/or optoacoustic and/or optothermal signals emanating from the tissue in response to irradiating the tissue with electromagnetic radiation, in particular in the near-infrared and/or visible spectral range, wherein the at least one irradiation unit and the at least one detection unit are located in the same half space above and/or adjacent to the irradiated tissue, and a processing unit configured i) to localize, based on the detected first signals, a location or region of interest on and/or within the tissue, at which at least one biomolecule of interest is present or expected, ii) to control the at least one irradiation unit to irradiate the location or region of interest on and/or within the tissue with electromagnetic radiation exhibiting a time-varying intensity and one or more wavelengths or wavelength ranges in the mid-infrared spectral range, iii) to control the at least one detection unit to detect second signals corresponding to optoacoustic and/or optothermal signals emanating from the irradiated location or region of interest on and/or within the tissue in response to irradiating the location or region of interest on and/or within the tissue with the electromagnetic radiation in the mid-infrared spectral range, and iv) to determine, based on the detected second signals, one or more parameters regarding a content and/or property of the at least one biomolecule, in particular metabolite, of interest at the location or region of interest on and/or within in the tissue.

US 2023/190145 A1 discloses a method of analyzing a material comprising at least one analyte, said method comprising a material status analyzing procedure, in which a present status of the material is analyzed.

CN 208808486 U discloses an opto-acoustic blood glucose detection device capable of automatically positioning blood vessels.

US 2008/262324 A1 discloses an approach of attaching and fixing a measurement head for a spectroscopic system to a variety of different parts of the skin of a patient.

SUMMARY OF THE INVENTION

It is thus an object of the present invention to improve the accuracy and reliability of the detection of an analyte in tissue of a human or animal subject by a method as set forth above.

This object is met by a device for detecting an analyte in tissue of a human or animal subject according to claim 1 and a method for detecting an analyte in tissue of a human or animal subject according to claim 9. Examples of the present invention are detailed in the dependent claims.

The device for detecting an analyte in tissue of a human or animal subject according to the invention comprises a measurement body, an excitation radiation source, a detection device and a controller. The measurement body has a contact surface suitable to be brought in contact with a surface of a skin of the subject, said contact permitting heat and/or pressure waves generated by absorption of excitation radiation in the tissue to be transferred to said measurement body. The excitation radiation source is configured for irradiating excitation radiation at a plurality of wavelengths into the tissue to be absorbed by the analyte contained therein. The detection device is configured to detect a physical response of the measurement body or of a component included therein to a heat and/or pressure wave received from the tissue upon absorption of the excitation radiation. The detection device is further configured to generate a response signal based on said detected physical response, said response signal being indicative of a degree of absorption of excitation radiation. The controller is configured to control the excitation radiation source to irradiate excitation radiation into the tissue to be absorbed by the analyte contained therein. The controller is further configured to control the detection device to detect said physical response and to generate a response signal indicative of the degree of absorption of said excitation radiation. The device further comprises means that are configured to move said contact surface and a propagation axis of the excitation radiation irradiated into the tissue transversally with respect to each other while the contact surface is in contact with the skin surface of the subject to adjust an irradiation position on the skin surface at which said excitation radiation is to be irradiated into the tissue.

The analyte is not particularly limited and may be any substance that absorbs excitation radiation at a plurality of analyte-characteristic wavelengths. Preferably, the analyte is a physiologically relevant substance that is present in tissue of humans and/or animals. The analyte may in particular be glucose.

The tissue may be any tissue in the human or animal body that can be irradiated by excitation radiation and from which heat and/or pressure waves may be transferred to the measurement body. The tissue may for example be sufficiently close to the skin or skin surface of the subject for the tissue to be reached by excitation radiation irradiated through the skin or skin surface of the subject and/or for heat and/or pressure waves generated by absorption of the excitation radiation in the tissue to propagate to the surface of the skin. The tissue may in particular be the skin of the subject. In one example, the analyte is glucose, in particular glucose present in an interstitial fluid of the skin, i.e. in a fluid present in a space between cells within the skin. While in the following explanations and in the description of the specific embodiments reference may be made to this embodiment, it is to be understood that the invention is not limited to this, and that it can be applied to other types of tissue and/or analytes.

The measurement body may be any body that (or a component thereof) exhibits a physical response to heat and/or pressure waves that can be detected with a detection device, e.g. as described above. The measurement body may be a solid body, in particular a transparent solid body. In some embodiments, the body may also comprise a fluid, which may e.g. be contained in a cavity or void within the measurement body and/or in a recess or cut-out in the measurement body (e.g. in the contact surface). The contact surface may be a surface of the measurement body that is exposed from the device, i.e. not covered by any other part of the device, such that it can be brought in contact with the skin surface of the subject. The contact surface may be a simply connected surface (i.e. without holes), but may also for example be a surface extending around a recess or cut-out in the measurement body (e.g. an edge or rim of the recess). The contact between the measurement body and the skin surface may be a thermal contact that permits the transfer of heat waves to the measurement body, e.g. through the contact surface, and/or may be a pressure transmitting contact that permits the transfer of pressure waves to the measurement body, e.g. through the contact surface and/or through an interface or plane surrounded by the contact surface (e.g. a "surface" or opening of a recess in the measurement body that is surrounded by the contact surface).

The excitation radiation source is configured to generate excitation radiation at a plurality of wavelengths ("excitation wavelengths"), in particular at a plurality of analyte-characteristic wavelengths. Said plurality of wavelengths or analyte-characteristic wavelengths may for example comprise between 2 and 50 wavelengths, in some examples between 5 and 20 wavelengths. The excitation radiation source may for example be a light source such as a laser that is configured to generate excitation radiation in the optical spectrum, for example in the infrared spectrum, in particular in the near-infrared and/or mid-infrared spectrum. For irradiating the excitation radiation generated by the excitation radiation source into the tissue, e.g. through the contact surface and/or through the measurement body, the device may comprise one or more optical elements such as waveguides, lenses and/or mirrors.

In some embodiments, said excitation radiation is generated using an array of lasers, in particular semiconductor lasers, further in particular quantum cascade lasers, each having a dedicated wavelength. Each of the lasers may have its own modulation device, or they may have a common modulation device and they may be controlled by a common or by individual control units. The lasers of an array may be optically aligned in a way that allows for using a common optical path for the excitation beam and they may for example radiate into the tissue through a common light waveguide. The laser array may be combined on a single semiconductor chip.

In some embodiments, said excitation radiation is generated using at least one tunable laser, in particular at least one tunable quantum cascade laser.

In a preferred embodiment, some or all of said excitation wavelengths are in a range of 5 μm to 13 μm, preferably 8 μm to 11 μm. In alternative embodiments, some or all of said excitation wavelengths are in a range

of 3 μm to 5 μm. This wavelength range is for example useful for detecting absorption of $CH_2$ and $CH_3$ vibrations in fatty acids.

The detection device may be any device that is configured to detect the physical response of the measurement body or of the component therein to heat and/or pressure waves and to generate a corresponding response signal, e.g. as detailed above. The detection device may comprise one or more detection elements such as a photodetector, an electrode, a temperature sensor and/or a microphone for detecting the physical response. The detection device may further comprise one or more probing sources such as a light source and/or a current or voltage source for generating a probing excitation such as a light beam and a current or voltage, respectively, that is to be detected by the one or more detection elements, e.g. to detect an effect of a heat and/or pressure wave on the probing excitation such as a deflection of the light beam. The response signal generated by the detection device may allow for quantifying the physical response of the measurement body and thereby the degree of absorption of excitation radiation in the tissue.

In some embodiments, said detection device comprises a light source for generating a detection light beam travelling through at least a portion of said measurement body or a component included in said measurement body, said physical response of the measurement body to heat received from said tissue upon absorption of said excitation radiation is a local change in the refractive index of said measurement body or said component, and said detection device is configured for detecting one or both of a change in the light path and a change in the phase of detection beam due to said change in refractive index and for generating a response signal indicative of said change in light path and/or phase of the detection beam.

In some embodiments, said measurement body is transparent for said detection light beam, said detection light beam is directed to be totally or partially reflected at a surface of said measurement body that is in contact with said skin surface (in particular at the contact surface), and wherein said detection device comprises a photodetector, in particular a position sensitive photodetector, capable of detecting a degree, in particular angle of deflection of said detection light beam due to said local change in refractive index.

In some embodiments, said detection device comprises an interferometric device allowing for assessing said change in phase of the detection beam and generating a response signal indicative of said change in phase.

In some embodiments, said measurement body or a component in said measurement body has electrical properties that change in response to a local change in temperature or a change in pressure associated therewith, and wherein said detection device comprises electrodes for capturing electrical signals representing said electrical properties.

The controller is configured to perform analyte measurements for detecting the analyte in said tissue, i.e. to control the excitation radiation source to irradiate excitation radiation, in particular excitation radiation at a plurality of analyte-characteristic wavelengths, into the tissue to be absorbed by the analyte contained therein and to control the detection device to detect said physical response and to generate a response signal indicative of the degree of absorption of said excitation radiation. The controller may be configured to control the excitation radiation source such that the intensity of said excitation radiation is time-modulated (e.g. pulsed) and/or such that different analyte-characteristic wavelengths that are irradiated one or both of simultaneously and sequentially.

The controller may be embodied in hardware, software or both. In particular, the controller may comprise one or more computers, processors, microcontrollers, FPGAs, ASICs and corresponding computer programs which when executed on the corresponding hardware provide the control functions described herein. In some examples, the controller may be a distributed system, for example comprising several control units that are in data communication with each other, of which some may be provided in a housing of the device, and others remote from the housing, but it can also be formed by a single control unit. To control components of the device such as the excitation radiation source and the detection device, the controller may be configured to generate digital and/or analog control signals for the respective component. In a preferred embodiment of the device, said controller is configured to control components of the device for carrying out a method for detecting an analyte in tissue of a human or animal subject according to any one of the embodiments described herein at least in part, in one example in its entirety (i.e. to execute all steps of said method).

The controller may further be configured to perform an analyzing step, for example as described above. The controller may in particular be configured to determine a measure of the concentration of the analyte in the tissue, e.g. the concentration itself or a quantity that has a known functional relation to the concentration (e.g. is proportional to the concentration), based on the response signal.

When the excitation radiation is radiated into the tissue, part of it will be absorbed along its light path, e.g. at different depths below the skin surface (e.g. in the skin), for example by the analyte contained within the tissue. In response to the absorption, a heat signal is generated, which diffuses back to the skin surface and is received by the measurement body. When the intensity of the excitation radiation is modulated, the tissue is heated by absorption in a time-dependent manner, leading to a temperature field that varies as a function of space and time and that is often referred to as a thermal wave. The term thermal "wave" is somewhat misleading, since the travel of heat through the material is not governed by a wave equation, but by a diffusion equation instead. This also means that the largest depth of the modulated absorption underneath the surface that can be detected by heat received by the measurement body at the surface is limited approximately by the so-called diffusion length $\mu_t$, which depends on the density $\rho$, the specific heat capacity $C_p$, and the thermal conductivity $k_t$ of the tissue as well as on the modulation frequency f of the excitation radiation:

$$\mu t(f) = \sqrt{\frac{k_t}{\rho \cdot C_p \cdot 2\,f}}$$

Since the interstitial fluid is only found in the tissue at a certain range below the surface of the skin, in order to generate response signals indicative of absorption in the interstitial fluid, the modulation frequency of the excitation radiation has to be indeed "sufficiently low" such that the diffusion length $\mu_t$ is long enough to cover the distance between regions with interstitial fluid and the skin surface. However, as explained above, the diffusion length depends on the physical properties of the tissue, namely density $\rho$, the specific heat capacity $C_p$, and the thermal conductivity kt , and the present applicant noticed that these properties will not only vary from person to person, but may also vary for the same person with time, depending for example on whether the skin is dry or moist, whether the skilled person has used hand moisture lotion, or the like. In other words, the thermal diffusion length as a function of frequency is a material/tissue status that for best results should be assessed at or close to the time of the analyte measurement procedure in a material/tissue status analyzing procedure, e.g. as described in WO 2021/233559 A1. Thereby, a modulation frequency may be determined that is "sufficiently low" such that the response signal reflects at least in part absorption of excitation radiation within the interstitial fluid.

Moreover, the depth below the surface at which the interstitial fluid resides is found to be dependent on the thickness of the stratum corneum, which again does not only vary from person to person, but also varies for the same person over time and also over the location of the skin surface where the measurement takes place. For example, if a person has recently done physical work with his or her hands, or has practiced a string instrument, the stratum corneum may be thicker than usual, meaning that the absorption has to be effected in deeper layers below the surface of the skin in order to cover the interstitial fluid. Again, the thickness of the stratum corneum is a "material/tissue status" that can be assessed in said material/tissue status analyzing procedure.

However, even if the thermal diffusion length is sufficiently long that the response signal reflects at least in part absorption of excitation radiation within the interstitial fluid, the response signal will also represent any absorption in upper layers of the skin, in particular the stratum corneum, and hence include a contribution that is not related to the glucose within the interstitial fluid. Indeed, this contribution from upper layers of the skin, in particular the stratum corneum, will be typically overrepresented in the response signal, since the intensity of the excitation radiation in the upper layers is higher than deeper into the skin, and since the absorption heat needs to travel only a shorter distance from the upper layers to reach the measurement body. In order to estimate this contribution of higher layers of the skin to the (first) response signal, at least one additional second response signal may be recorded in response to excitation radiation with a second modulation frequency, which is higher than the other (first) modulation frequency, and hence leads to a shorter diffusion length and therefore represents absorption predominantly in the upper layers of the skin above the region where the interstitial fluid resides. Then, response signals corresponding to said modulation frequencies, or quantities derived therefrom, are mathematically combined to yield information more specifically indicative of the absorption in the interstitial fluid. There are different ways of mathematical combining these response signals, and this aspect of the invention is not limited to any single one of them. For example, the second response signal can be multiplied with a normalizing factor that is obtained e.g. based on reference measurements with excitation wavelength(s) for which glucose absorption is negligible, and then the product may be subtracted from the first response signal.

The device comprises means configured to move said contact surface and a propagation axis of the excitation radiation irradiated into the tissue with respect to (i.e. relative to) each other, i.e. such that the contact surface moves or is displaced relative to the propagation axis and/or vice-versa, e.g. by moving one or both of the contact surface and the propagation axis of the excitation radiation. The propagation axis may for example correspond to a propagation direction or direction of travel of the excitation radiation (e.g. of an excitation light beam) at the contact surface. The device may comprise an optical system configured to direct excitation radiation generated by the excitation radiation source into the tissue and the propagation axis may correspond to an optical axis of said optical system.

The device may in particular comprise actuated means, for example one or more actuators and/or one or more deformable members as detailed below, that are configured to move the contact surface, e.g. by moving the entire measurement body or one or more components thereof. The actuated means are configured to move the contact surface with respect to (i.e. relative to) the propagation axis of the excitation radiation, i.e. such that the contact surface moves or is displaced relative to the propagation axis. The actuated means may in particular be configured to move the contact surface such that the excitation radiation source and/or the propagation axis of the excitation radiation remain unchanged (for example do not move within the device, e.g.

relative to other components of the device).

The actuated means are configured to move the contact surface transversally with respect to the propagation axis, in particular perpendicular or substantially perpendicular with respect to the propagation axis. In the context of this disclosure, "moving transversally with respect to the propagation axis" is to be understood as referring to any motion that at least has a non-zero transverse component (e.g. projection) in a direction perpendicular to the propagation axis with said transverse component being sizeable in comparison with (i.e. at least 50%, preferably at least 75%, most preferably at least 100% of) the longitudinal component (e.g. projection) of the motion in the direction parallel to the propagation axis. In other words, the motion of the contact surface does not necessarily have to be exactly (or only) perpendicular to the propagation axis, but may for example also be at an angle to the plane perpendicular to the propagation axis (i.e. at an angle different from 90° to the propagation axis). Preferably, the actuated means are configured to move the contact surface parallel or substantially parallel to the contact surface.

The actuated means may be configured to move the contact surface in a (first) direction that is at an angle to the propagation axis. In some examples, the actuated means may be configured to move the contact surface in the first direction and in a second direction different from the first direction independently (i.e. such that the contact surface may be moved only along one of the directions, but not along the other direction and/or vice-versa). The actuated means may in particular be configured to also move the contact surface longitudinally with respect to (e.g. parallel or substantially parallel to) the propagation axis as detailed below.

The first direction may for example be at an angle between 30° and 150°, in some examples between 45° and 135° to the propagation axis. Preferably, the first direction is perpendicular or substantially perpendicular to the propagation axis. In the context of this disclosure, two directions (or axes or surfaces) may for example be considered to be substantially perpendicular to each other if the two directions (or axes or surfaces) are at an angle between 75° and 105°, preferably between 80° and 100°, in some examples between 85° and 95° to each other. The expression "substantially perpendicular" is in particular to be understood as encompassing tilt angles at which optical elements are typically arranged relative to an optical axis to avoid back-reflections along the optical axis. Similarly, two directions (or axes or surfaces) may for example be considered to be substantially parallel to each other if the two directions (or axes or surfaces) are at an angle between -15° and 15°, preferably between -10° and 10°, in some example between -5° and 5° to each other.

The actuated means are configured to move the contact surface while the contact surface is in contact with the surface of the skin of the subject. The present inventors have observed that adhesion and/or friction between the contact surface and the surface of the skin is sufficiently strong such that the portion of the skin that is in contact with the contact surface follows a motion of the contact surface at least in part, in most cases perfectly or almost perfectly (e.g. such that there is no or almost no relative displacement between the contact surface and the respective portion of the surface of the skin). By moving the contact surface transversally with respect to the propagation axis, the skin (or at least the surface thereof) of the subject may thus be moved (displaced) transversally relative to the propagation axis, e.g. such that the position at which the excitation radiation is incident on the surface of the skin changes.

This allows for changing the irradiation position on the surface of the skin at which excitation radiation is irradiated into the tissue, e.g. the portion of the skin surface through which the excitation radiation is irradiated into the tissue (i.e. the region or spot on the skin surface that is illuminated by the excitation radiation, referred to as the "irradiation region" in the following). Thereby, the portion of the skin or skin surface through which heat and/or pressure waves generated by absorption of excitation radiation in the tissue are transferred to the measurement body ("transfer region") may also be changed. In other words, the region or spot on the surface of the skin at which (e.g. in and/or below which) an analyte measurement is performed (referred to as "measurement region" or "measurement spot" in the following) may be changed, for example using the method according to the invention described herein. Preferably, the actuated means are controlled automatically, e.g. by the controller, such that the irradiation position (and thus the measurement region) may be moved automatically without user intervention. This may facilitate avoiding irradiation positions or measurement regions that are unsuitable or less suitable for analyte measurements, for example due to an insufficient transmission of heat and/or pressure waves to the measurement body and/or an insufficient transmission of excitation radiation into the tissue.

Instead of or in addition to moving the contact surface with respect to the propagation axis of the excitation radiation, the propagation axis of the excitation radiation may be moved with respect to the contact surface to adjust the irradiation position on the skin surface. For this, the device may comprise deflection means configured to move the propagation axis with respect to the contact surface while the contact surface is in contact with the skin surface of the subject. Said deflection means may for example comprise optical deflection elements such as a moveable (e.g. tiltable) optical element (for example a moveable mirror such as a piezoactuated mirror), an adjustable refractive element (e.g. a lens with an adjustable focal length) and/or an acousto-optic modulator. While moving the contact surface may allow for adjusting the irradiation position without altering the propagation axis of the excitation radiation relative to the detection device (e.g. relative to a detection light beam), the present inventors have observed that the displacements required for finding suitable irradiation positions typically are sufficiently small so as to still allow for reliable measurements even when the propagation axis of the excitation radiation is moved instead, which may be easier to implement in some cases. In some examples, both approaches may be combined, e.g. to allow for moving the contact surface and the propagation axis transversally with respect to each other in two directions (e.g. in a first direction by moving the contact surface and in a second direction by moving the propagation axis).

Said deflection means are configured to move the propagation axis of the excitation radiation transversally with respect to the contact surface, in particular parallel or substantially parallel with respect to the contact surface. In the context of this disclosure, "moving the propagation axis of the excitation radiation transversally with respect to the contact surface" is to be understood as referring to any motion or adjustment of the propagation axis (i.e. of the propagation or light path) of the excitation radiation that results in a change in the position on the contact surface at which the excitation radiation is incident on the contact surface (i.e. in the position on the contact surface at which the propagation axis intersects with the contact surface). Moving the propagation axis may comprise tilting and/or translating (i.e. parallel displacing) the propagation axis.

The controller is configured to determine a response/contact measure, wherein the response/contact measure may indicate a suitability of the current irradiation position and/or measurement region for analyte measurements. The response/contact measure indicates (e.g. characterizes or quantifies) a degree to which said measurement body exhibits a physical response upon irradiating excitation radiation into the tissue to be absorbed therein and/or indicates a degree to which said contact permits heat and/or pressure waves generated by absorption of excitation radiation in the tissue to be transferred to said measurement body. The response/contact measure may for example indicate (or be) an amplitude or magnitude of the physical response and/or of the response signal, e.g. upon irradiation of excitation radiation into the tissue at one or more of said plurality of wavelengths. Additionally or alternatively, the response/contact measure may for example indicate a transmission efficiency (e.g. an attenuation) of said heat and/or pressure waves to the measurement body, for example qualitatively (e.g. "good" or "poor") and/or quantitatively. In some examples, the response/contact measure may be or comprise a parameter or quantity that is determined as part of an analyte measurement, for example the amplitude of the response signal at one or more analyte-characteristic wavelengths and/or the difference in amplitude of the response signal between two analyte-characteristic wavelengths.

The controller may be configured to control said means (e.g. said actuated means and/or said deflection means) to move the contact surface transversally with respect to the propagation axis of the excitation radiation and/or vice-versa, in particular based on said response/-contact measure. The controller may for example be configured to determine (e.g. decide) whether or not to move the contact surface and the propagation axis with respect to each other based on the response/contact measure, e.g. to move the contact surface and the propagation axis with respect to each other in response to determining that the response/contact measure does not meet a (first) response/contact threshold and to not move the contact surface and the propagation axis with respect to each other in response to determining that the response/contact measure does meet said response/contact threshold. Additionally or alternatively, the controller may be configured to determine a distance by which to move the contact surface and the propagation axis with respect to each other based on the response/contact measure.

The controller may be configured to determine (e.g. decide) whether or not to perform and/or discard an analyte measurement based on said response/contact measure, e.g. to perform an analyte measurement in response to determining that the response/contact measure meets a (second) response/contact threshold and to not perform or to discard an analyte measurement in response to determining that the response/contact measure does not meet said response/contact threshold. In some examples, the second response/contact threshold may be the same as the first response/contact threshold.

The controller may be configured to determine the response/contact measure based on a response signal generated by said detection device. The controller may for example be configured to determine a measure for an amplitude or magnitude of the response signal (e.g. the amplitude or magnitude itself). The controller may be configured to determine the response/contact measure by controlling the excitation radiation source to irradiate excitation radiation (e.g. at a current irradiation position) into the tissue to be absorbed therein and controlling the detection device to detect said physical response and to generate a response signal indicative of the degree of absorption of said excitation radiation. Preferably, the excitation radiation is irradiated into the tissue at one or more wavelengths at which the degree of absorption of said excitation radiation is substantially independent of a concentration of the analyte in the tissue. Additionally or alternatively, the excitation radiation may also be irradiated into the tissue at one or more analyte-characteristic wavelengths (determining the response/contact measure may e.g. be or comprise performing an analyte measurement). In some examples, the controller may additionally or alternatively be configured to determine the response/contact measure based on a response signal that is generated by said detection device without irradiating excitation radiation into the tissue, for example a response signal generated in response to heat and/or pressure waves transferred to the measurement body when bringing the contact surface in contact with the skin surface of the subject and/or a response signal associated with a deflection, reflection, scattering and/or absorption of a detection light beam at the contact surface and/or the skin surface when the contact surface is in contact with the skin surface.

As used herein, the degree of absorption of said excitation radiation may for example be considered to be substantially independent of the concentration of the analyte if the absorptivity of the analyte in said tissue is less than 5%, preferably less than 2%, in some examples less than 1%, in one example less than 0.5% of the highest absorptivity at any of the analyte-characteristic wavelengths relied on in the analyte measurement and/or less than 30%, preferably less than 20%, in some examples less than 10%, in one example less than 5% of the lowest absorptivity at any of the analyte-characteristic wavelengths relied on in the analyte measurement.

In some examples, the device may comprise an auxiliary sensor that is configured to generate a sensor signal that is indicative of the degree to which said contact permits heat and/or pressure waves generated by absorption of excitation radiation in the tissue to be transferred to said measurement body. The controller may be configured to determine the response/contact measure based on the sensor signal generated by the auxiliary sensor, e.g. instead of or in addition to a response signal generated by the detection device.

As used herein, an auxiliary sensor may for example be a sensor that is per se unrelated to the measurement apparatus for measuring the analyte absorption. The auxiliary sensor may for example be or comprise an optical sensor. The optical sensor may be configured to generate a sensor signal that is indicative of a degree of reflection, scattering, deflection and/or absorption of light at the contact surface and/or at the skin surface in contact with the contact surface. In one example, the optical sensor may comprise an imaging device such as a camera for taking images of the contact surface. Additionally or alternatively, the auxiliary sensor may be or comprise an electrical sensor, in particular a capacitive sensor.

In some embodiments, the auxiliary sensor may be configured to detect microstructural features (e.g. furrows and/or ridges such as friction ridges) on the skin surface and/or air pockets between the contact surface and the skin surface. The auxiliary sensor may for example be configured to determine a pattern of microstructural features on the skin surface (e.g. similar to a fingerprint sensor) and/or of air pockets, in particular to determine the position of said microstructural features and/or air pockets relative to a current irradiation position and/or a current measurement region and/or relative to the propagation axis of the excitation radiation. Based on the detected microstructural features and/or air pockets (e.g. based on their position relative to the current irradiation position and/or measurement region), the degree to which said contact permits heat and/or pressure waves generated by absorption of excitation radiation in the tissue to be transferred to said measurement body may be determined (e.g. estimated qualitatively and/or quantitatively). For example, if an air pocket is detected along or in the vicinity of the propagation axis, this may indicate a reduced transmission efficiency of heat and/or pressure waves generated by absorption of excitation radiation in the tissue to the measurement body ("poor contact").

As mentioned above, the device may comprise actuated means that are configured to move the contact surface transversally with respect to the propagation axis of the excitation radiation while the contact surface is in contact with the skin surface of the subject. The actuated means may comprise one or more actuators, wherein said one or more actuators may for example comprise one or more of a piezo element (e.g. an element comprising or consisting of a piezoelectric material), an electric actuator (e.g. an actuator comprising an electric motor), a magnetic actuator (e.g. an actuator configured to generate or switch a magnetic field to move a magnetic object or an electromagnet) and a fluid pump (e.g. for inflating and deflating an inflatable body).

Additionally or alternatively, the actuated means may comprise one or more deformable members configured to be deformed, extended and/or retracted (e.g. by an actuator, which may or may not be part of the device according to the invention). Said one or more deformable members may for example comprise one or more of an inflatable body (e.g. a body enclosing a volume that is configured to receive a fluid such as air to be expanded from a compressed state to an expanded state) and/or a pneumatically and/or hydraulically extendable member (e.g. a hydraulic or pneumatic cylinder or a pneumatically or hydraulically movable shaft or piston).

The actuated means may be configured to move said contact surface by moving the entire measurement body or a component thereof, e.g. a member such as a wall, slab or membrane forming the contact surface. The actuated means may be configured to move the contact surface by at least 100 $\mu$m (e.g. by up to a distance between 100 $\mu$m and 5 mm), in some examples by at least 500 $\mu$m, preferably by at least 1 mm, in one example by at least 2 mm perpendicular to the propagation axis of the excitation radiation (e.g. such that the contact surface is displaced by the corresponding distance in a direction perpendicular to the propagation axis).

The detection device may comprise a light source for generating a detection light beam that travels through at least a portion of the measurement body or of a component included therein, e.g. for detecting a change in the light path and/or in the phase of said detection light beam as described above. The actuated means may be configured to move the contact surface in a direction that is perpendicular or substantially perpendicular to both the propagation axis of said excitation radiation irradiated into the tissue and a propagation axis of the detection light beam (e.g. a propagation axis of the detection light beam at the contact surface or when entering or being incident on the measurement body or said component included therein). This may for example allow for moving the contact surface without altering the propagation of the excitation radiation and/or of the detection light beam.

In some examples, the measurement body may comprise a prism, in particular a trapezoidal prism. Said prism may have a first input facet that is perpendicular or substantially perpendicular to the propagation axis of said excitation radiation and/or a second input facet that is perpendicular or substantially perpendicular to the propagation axis of said detection light beam. The actuated means may be configured to move the contact surface and the prism in a direction that is parallel or substantially parallel to one or both of the first and second input facets. This may for example allow for moving the contact surface without altering the refraction of the excitation radiation and/or of the detection light beam at the first and second input facet, respectively.

The actuated means may also be configured to move the contact surface longitudinally with respect to the propagation axis of said excitation radiation, in particular parallel or substantially parallel to the propagation axis (e.g. in a (second) direction that is parallel or substantially parallel to said propagation axis). The actuated means may in particular be configured to move the contact surface longitudinally with respect to the propagation axis of said excitation radiation for temporarily increasing a contact pressure between the measurement body and the surface of the skin of the subject, e.g. to establish and maintain close contact for performing an analyte measurement, for fixing the skin surface of the subject transversally relative to the propagation axis and/or for moving the skin surface of the subject transversally relative to the propagation axis by moving the contact surface (for example for fixing the skin surface relative to, e.g. on, the contact surface). The actuated means may for example be configured to temporarily increase the contact pressure to between 0.1 N/cm$^2$ and 100 N/cm$^2$, preferably to between 1 N/cm$^2$ and 10 N/cm$^2$.

The device may be configured for use with or on human subjects (e.g. a user or a patient), but may also be adapted for use with or on animal subjects, e.g. by adjusting the physical dimensions and/or the shape of the device accordingly. The animal subjects may be mammals, in particular larger mammals (e.g. mammals with a body size on the same order of magnitude as humans) such as cats, dogs, pigs and cows.

In some examples, the device may be a wearable device that is configured to be worn by the subject by securing or fastening the wearable device to a body part of the subject. The wearable device may for example comprise a holding portion configured to be arranged around the body part of the subject so as to extend circumferentially around the body part at least in part. The physical dimensions and the weight of the wearable device may be such that the wearable device can be carried by the subject, e.g. such that the wearable device can be worn by the subject over extended periods of time (e.g. for more than one hour) or permanently. In some embodiments, the wearable device can for example be a wristwatch (in particular a smartwatch), a fitness tracker or a chest-strap monitor.

The present invention further provides a method for detecting an analyte in tissue of a human or animal subject using a device according to any one of the embodiments disclosed herein. The method is to be performed while the contact surface of the measurement body of said device is in contact with a surface of the skin of the subject. The method comprises moving said surface of the skin of the subject and the propagation axis of the excitation radiation transversally with respect to each other by moving one or both of the contact surface and the propagation axis of the excitation radiation using said means (e.g. said actuated means and/or said deflection means) with the contact surface being in contact with said surface of the skin of the subject to adjust an irradiation position on said surface of the skin at which said excitation radiation is to be irradiated into the tissue. An analyte measurement is performed by irradiating excitation radiation at said adjusted irradiation position into the tissue to be absorbed by the analyte contained therein using the excitation radiation source, detecting the physical response of the measurement body or a component included therein with the detection device and generating a response signal indicative of the degree of absorption of said excitation radiation using the detection device.

In some embodiments, the method may further comprise determining a response/contact measure, said response/contact measure indicating one or both of a degree to which said measurement body exhibits a physical response upon irradiating excitation radiation into the tissue to be absorbed therein and a degree to which said contact permits heat and/or pressure waves generated by absorption of excitation radiation in the tissue to be transferred to said measurement body. The response/contact measure may indicate a suitability of the current irradiation position (e.g. of a current measurement region/spot at which an analyte measurement is to be performed that is associated with the current irradiation position) for analyte measurements, e.g. as described above for the device according to the invention. The response/contact measure may be determined as described above for the device according to the invention, for example based on a response signal generated by the detection device and/or based on a sensor signal generated by an auxiliary sensor. The response/contact measure may be determined prior to or after moving the surface of the skin and the propagation axis of the excitation radiation with respect to each other.

The surface of the skin of the subject and the propagation axis of the excitation radiation may be moved transversally with respect to each other by moving the skin surface of the subject transversally with respect to the propagation axis of the excitation radiation by moving the contact surface using said actuated means. In some examples, the contact surface may be moved such that the propagation axis of the excitation radiation remains unchanged (e.g. does not move within the device). Additionally or alternatively, the propagation axis of the excitation radiation may be moved transversally with respect to the skin surface using said deflection means in some examples.

The skin surface of the subject and the propagation axis of the excitation radiation may be moved transversally with respect to each other based on said response/contact measure. This may in particular comprise determining (e.g. deciding) whether or not to move the contact surface and the propagation axis with respect to each other based on the response/contact measure. The method may for example comprise comparing the response/contact measure with a (first) response/contact threshold. Said surface of the skin of the subject and the propagation axis may be moved with respect to each other in response to determining that the response/contact measure does not meet the response/contact threshold (i.e. if or in case the response/contact measure does not meet the response/contact threshold). On the other hand, if the response/contact measure meets the response/contact threshold, the skin surface of the subject and the propagation axis may not be moved with respect to each other but the method may e.g. directly proceed to performing the analyte measurement.

Additionally or alternatively, the method may also comprise determining a distance by which to move the contact surface and the propagation axis of the excitation radiation with respect to each other (and thus at least in part the skin surface and the propagation axis with respect to each other) based on said response/contact measure. For example, the contact surface and the propagation axis may be moved with respect to each other by larger distance in case the response/contact measure indicates a lower suitability of the current irradiation position and/or of the current measurement region/spot (e.g. a smaller physical response or a lower transmission efficiency of heat and/or pressure waves) and by a smaller distance in case the response/contact measure indicates a higher suitability of the current irradiation position and/or of the current measurement region/spot (e.g. a larger physical response or a higher transmission efficiency of heat and/or pressure waves).

By moving the surface of the skin of the subject transversally with respect to the propagation axis of the excitation radiation (i.e. displacing the skin surface relative to the propagation axis) and/or vice-versa, the irradiation position on the skin surface (for example the irradiation region, i.e. the portion of the skin surface through which the excitation radiation is to be irradiated into the tissue) may be adjusted, for example changed or moved to a different position on the skin surface. Thereby, the measurement region/spot on the skin surface (i.e. the region or spot on the skin surface at which, e.g. in and/or below which, an analyte measurement is to be performed) may be adjusted, for example to move to a more suitable measurement region or spot on the skin surface. The surface of the skin of the subject and the propagation axis may be moved with respect to each other to adjust the portion of the skin through which heat and/or pressure waves generated by absorption of excitation radiation in the tissue are to be transferred to the measurement body.

At the adjusted measurement region (which is associated with the adjusted irradiation position), the analyte measurement is then performed, e.g. after moving the skin surface of the subject and the propagation axis with respect to each other based on the response/contact measure (for example after moving the skin surface and the propagation axis with respect to each other or after determining that the skin surface and the propagation axis are not to be moved with respect to each other). For this, the excitation radiation is irradiated into the tissue at the adjusted irradiation position (e.g. through the measurement region or a part thereof). The excitation radiation may be irradiated at one or more analyte-characteristic wavelengths, for example as described above. Heat and/or pressure waves generated by absorption of excitation radiation in the tissue may be transferred to the measurement body at the measurement region (e.g. through the measurement region or a part thereof). The physical response and the response signal may be detected and generated, respectively, as described above. In some examples, the method may comprise determining a measure of the concentration of the analyte in the tissue, in particular the concentration itself, based on a response signal, e.g. the response signal generated when performing the analyte measurement at the adjusted irradiation position/measurement region.

In some examples, the method may comprise optimizing the response/contact measure by moving the skin surface of the subject and the propagation axis of the excitation radiation transversally with respect to each other, e.g. to find an optimized adjusted irradiation position and/or measurement region. This may comprise repeatedly moving the skin surface of the subject and the propagation axis with respect to each other (e.g. to adjust/move the irradiation position/measurement region to a current irradiation position/measurement region) and determining a response/contact measure (e.g. for the current irradiation position/measurement region). For example, the skin surface of the subject and the propagation axis may be moved with respect to each other until the response/contact measure reaches an (e.g. local) maximum. Optimizing the response/contact measure may comprise scanning the irradiation position/measurement region along one or two axes, e.g. by moving the skin surface of the subject along one or two axes perpendicular to the propagation axis of the excitation radiation (and/or vice-versa).

The response/contact measure may be determined repeatedly and the method may further comprise performing an active feedback control based on said response/contact measure to adjust said irradiation position and/or measurement region by moving the skin surface of the subject and the propagation axis of the excitation radiation transversally with respect to each other using said means. For example, the response/contact measure may be monitored and compared to the response/contact threshold. Whenever the response/contact measure fails to meet the response/contact threshold, the irradiation position/measurement region may be adjusted, e.g. until the response/contact measure meets the response/contact threshold again or until an optimized response/contact measure is found.

The response/contact measure may be determined based on a response signal generated by the detection device. Determining the response/contact measure may for example comprise controlling the excitation radiation source to irradiate excitation radiation at a current irradiation position into the tissue to be absorbed therein, controlling the detection device to detect said physical response and to generate a response signal indicative of the degree of absorption of said excitation radiation and determining the response/contact measure based on said response signal. Preferably, the excitation radiation is irradiated into the tissue at one or more wavelengths at which the degree of absorption of said excitation radiation is substantially independent of a concentration of the analyte in the tissue (e.g. at one or more characteristic wavelengths of another substance, in particular water-characteristic wavelengths). In other examples, the excitation radiation may additionally or alternatively be irradiated into the tissue at one or more analyte-characteristic wavelengths. Determining the response/contact measure may for example be or comprise performing an analyte measurement (or vice-versa). Determining the response/contact measure may thus be (or comprise) a first analyte measurement and the analyte measurement performed after moving the skin surface and the propagation axis with respect to each other (e.g. based on said response/contact measure) may be a second analyte measurement. The response/contact measure may be determined based on the response signal obtained from the first analyte measurement.

In some examples, an analyte measurement may also be performed prior to moving the skin surface of the subject and the propagation axis with respect to each other, e.g. to probe a different irradiation position and/or to determine the response/contact measure. In other words, the method may comprise performing a first analyte measurement before and a second analyte measurement after moving the skin surface of the subject and the propagation axis with respect to each other. The first analyte measurement may be performed at an initial irradiation position/measurement region, e.g. to determine a response/contact measure associated with the initial irradiation position and/or the initial measurement region. The method may comprise performing a plurality of analyte measurements, for example one or more analyte measurements at the initial irradiation position/measurement region (e.g. for determining the response/contact measure) and/or one or more analyte measurements at the adjusted irradiation position/measurement region after moving the skin surface based on the response/contact measure. Depending on whether the skin surface of the subject is moved or not, the adjusted irradiation position/measurement region may be different from the initial irradiation position/measurement region or may be the same as the initial irradiation position/measurement region.

In some examples, the method may comprise repeatedly performing analyte measurements and moving the skin surface of the subject and the propagation axis of the excitation radiation transversally with respect to each other between said analyte measurements (e.g. between some or all subsequent analyte measurements). Thereby, a plurality of irradiation positions may be sampled or scanned. The skin surface and the propagation axis may be moved with respect to each other in one or more directions, for example to perform a one-dimensional or two-dimensional scan of the irradiation position. In some embodiments, the method may comprise determining whether to discard an analyte measurement (e.g. the first and/or second measurements) based on a response/contact measure associated with the respective analyte measurement (e.g. determined as part of the respective analyte measurement and/or associated with (e.g. determined at) an irradiation position used for the respective analyte measurement). Additionally or alternatively, the method may also comprise averaging a plurality of analyte measurements (e.g. the response signals and/or other results such as a concentration or concentration measure), e.g. nondiscarded analyte measurements and/or some or all of the repeatedly performed analyte measurements.

In some examples, the response/contact measure may be or comprise a parameter or quantity that is any way determined as part of an analyte measurement, for example the amplitude of the response signal at one or more analyte-characteristic wavelengths and/or the difference in amplitude of the response signal between two analyte-characteristic wavelengths. In other words, performing an analyte measurement may intrinsically comprise or amount to the determination of a response/contact measure, which may for example be used to determine whether or not to discard the corresponding analyte measurement. Accordingly, the first analyte measurement may be performed in the same way as the second analyte measurement (i.e. may not comprise any additional steps that are not performed in the second analyte measurement or other analyte measurements, in particular not the determination of a separate response/contact measure that is not also determined as part of the second analyte measurement or other analyte measurements). In other examples, the determination of the response/contact measure and/or the first analyte measurement may be different from the second analyte measurement, which may e.g. not comprise the determination of a corresponding response/contact measure.

The controller of the device according to the invention may be configured to execute a method for detecting an analyte in tissue of a human or animal subject according to any one of the embodiments disclosed herein at least in part, preferably in its entirety (i.e. to execute some or all steps of the respective method). For this, the controller may be configured to control the excitation radiation source, the detection device and/or the actuated means accordingly.

## SHORT DESCRIPTION OF THE FIGURES

In the following, a detailed description of the invention and exemplary embodiments thereof is given with reference to the figures. The figures show schematic illustrations of

Fig. 1: the measurement principle underlying embodiments of the invention;

Fig. 2: the absorption spectrum of glucose in water, with the water background subtracted;

Fig. 3: a sectional view of an apparatus suitable for carrying out embodiments of the invention relying on response signals based on a deflection of a detection light beam;

Fig. 4: results of a Clarke's error grid analysis obtained with an apparatus of the type shown in Fig. 3;

Fig. 5: an apparatus suitable for carrying out embodiments of the invention relying on response signals based on piezoelectric responses to heat and/or pressure waves received from the tissue subjected to the analysis;

Fig. 6: an apparatus suitable for carrying out embodiments of the invention relying on response signals based on interferometrically detected phase changes in a detection light beam;

Fig. 7: a device for detecting an analyte in tissue of a human or animal subject according to an exemplary embodiment of the invention;

Fig. 8a: a microstructure on a surface of the skin of a subject;

Fig. 8b: the amplitude of a response signal as a function of the position of an irradiation position on the surface of the skin of the subject;

Fig. 9a, 9b: a device for detecting an analyte in tissue of a human or animal subject according to another exemplary embodiment of the invention in top view and side view, respectively;

Fig. 10: a flow chart of a method for detecting an analyte in tissue of a human or animal subject according to an exemplary embodiment of the invention;

Fig. 11: a flow chart of a method for detecting an analyte in tissue of a human or animal subject according to another exemplary embodiment of the invention; and

Fig. 12: a device for detecting an analyte in tissue of a human or animal subject comprising deflection means according to an exemplary embodiment of the invention.

## DESCRIPTION OF THE PREFERRED EMBODIMENTS

It is to be understood that both the foregoing general description and the following description are exemplary and explanatory only and are not restrictive of the methods and devices described herein. In this application, the use of the singular may include the plural unless specifically state otherwise. Also, the use of "or" means "and/or" where applicable or unless stated otherwise. Those of ordinary skill in the art will realize that the following description is illustrative only and is not intended to be in any way limiting. Other embodiments will readily suggest themselves to such skilled persons having the benefit of this disclosure. Reference will now be made in detail to various implementations of the example embodiments as illustrated in the accompanying drawings. The same reference signs will be used to the extent possible throughout the drawings and the following description to refer to the same or like items.

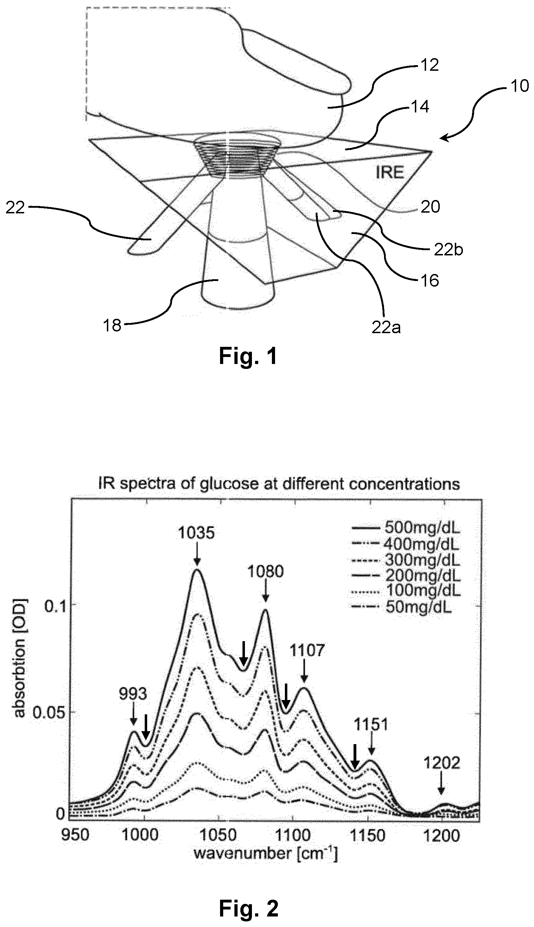

Fig. 1 is a schematic illustration of the measurement principle underlying the analyte measurement procedure summarized above and described in more detail in the following. While the device and method of the invention are suitable for analyzing various types of tissue of both human and animal subjects comprising at least one analyte, the following description will focus on specific embodiments, where the tissue is the skin of a patient and the analyte is glucose within the interstitial fluid of the skin. It is to be understood that all details and explanations given in the following with specific reference to glucose measurement are considered in relation to other types of tissue and analytes as well, where applicable, without explicit mention in the following.

In the illustration of Fig. 1, a fingertip 12 of a user is brought in thermal contact with a contact surface 14 of a measurement body 16. In an alternative implementation, which is not shown, the fingertip may be coupled acoustically to the measurement body through an acoustic cell which may include a hollow space filled with a liquid or gas allowing for a transfer of pressure waves to the measurement body.

An excitation beam 18 is irradiated to the measurement body 16 through air or through a waveguide (not shown) and then through the measurement body 16 and into the skin at the fingertip 12. In order to determine the concentration of the glucose in the skin, in particular in the interstitial fluid of the skin, various wavelengths of the excitation radiation 18 are chosen one after the other or at least partially at the same time for absorption measurement, such that from the measured absorption values the concentration of the glucose can be determined.

In Fig. 2, absorption spectra are shown for different concentrations of glucose in water, wherein the contribution of the absorption by water has been subtracted. As is seen therein, the glucose molecule has several characteristic absorption peaks in the mid infrared region at wave numbers ranging between 993 cm$^{-1}$ and 1202 cm$^{-1}$, corresponding to wavelengths ranging from 10.07 $\mu$m to 8.32 $\mu$m, respectively. In between adjacent absorption peaks, local absorption minima are seen, which are indicated by vertical arrows without wave numbers in Fig. 2. As is apparent from Fig. 2, particularly the difference in absorption at the absorption peaks and the local absorption minima are characteristic for the glucose concentration. Accordingly, in order to be able to determine the glucose concentration, it is preferable to measure the absorption at some or all of the absorption peaks and at some or all of the local absorption minima and potentially also at some point between the maxima and minima. These wavelengths are referred to as a "analyte(glucose)-characteristic wavelengths" herein. Note, however, that the absorptivity at the lowest local minimum at 1140 cm-1 is still 18% of the absorptivity at the highest peak at 1035 cm-1 in the relevant part of the spectrum. Accordingly, the absorption at any of these wavelengths will noticeably depend on the concentration of the glucose, such that these wavelengths are characteristic for the glucose, i.e. "glucose-characteristic wavelengths". In contrast to this, at about 1180 cm-1, the absorptivity is practically zero, and hence a global rather than a local minimum, and this wavelength is obviously not characteristic for the glucose (but may e.g. be used to determine a response/contact measure). While wavelengths exactly at the absorption peaks or local absorption minima are the preferred choices for the glucose-characteristic wavelengths, wavelengths close to the peaks/local minima but in an individually defined distance from them may also be used. Accordingly, as understood herein, "analyte-characteristic wavelengths" are also wavelengths where the difference in absorption to that at the closest absorption peak or the closest local absorption minimum is less than 30%, preferably less than 20% of the difference in absorption between the closest absorption peak and closest local absorption minimum.

The intensity of the excitation beam 18 is time modulated with a certain frequency f, such that the excitation radiation, in this case excitation light, has alternating intervals of high intensity and low or even vanishing intensity. Without wishing to limit the modulation to any particular waveform, high intensity intervals are referred to as "excitation light pulses" in the following. During the excitation light pulses, excitation light having the glucose-characteristic wavelength will be absorbed, such that the radiation energy will be converted to heat. Since the glucose molecules relax from the excited state within approximately 10$^{-12}$ s, the generation of a corresponding heat pulse and/or pressure wave can be regarded as occurring instantaneously for all practical purposes.

Accordingly, along with the excitation light pulses, local heat pulses are generated at the absorption site, leading to a temperature field that varies as a function of space and time and that could be referred to as a thermal wave. As was explained above, the term thermal "wave" is somewhat misleading, since the travel of heat through the material is not governed by a wave equation, but by a diffusion equation instead. However, the notion of a "heat wave" is correct at least to the extent that heat pulses propagate from within the skin to the surface 14 of the measurement body 16 and into the measurement body 16 similarly to what one is used to from wave propagation. A thermal gradient 20 that is caused by such a heat pulse is schematically shown in Fig. 1.

The heat received by the measurement body 16 from the skin of the fingertip 12 causes a physical response that can be detected with one of various possible detection devices which are devised for generating a response signal based on the physical response, wherein this response signal is indicative of the degree of absorption of the excitation light. Various ways of detecting the physical response and generating suitable response signals will be described below.

However, irrespective of the precise way of detecting the physical response, it is worth noting that the maximum depth underneath the surface of the skin in which the absorption can be detected by means of heat pulses travelling to the measurement body 16 is found to be limited to a good approximation by the thermal diffusion length $\mu_t$ of the skin, which is defined as

$$\mu t(f) = \sqrt{\frac{k_t}{\rho \cdot C_p \cdot 2\, f}}$$

and which depends on the density $\rho$, the specific heat capacity $C_p$, and the thermal conductivity $k_t$ of the tissue as well as on the modulation frequency f of the excitation light. In other words, by selecting the modulation frequency f, a depth can be defined up to which any absorption of the excitation light is reflected in the heat pulses received at the measurement body 16.

With reference again to Fig. 1, in the embodiment shown, the physical response to the absorption heat received from the skin is a change in refractive index in an area close to the surface 14 of the measurement body 16 where the heat gradient 20 is transiently formed.

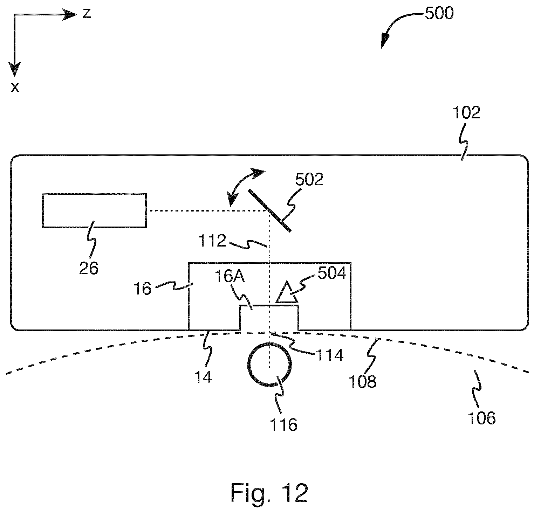

This local change in refractive index forms what could be regarded as a thermal lens that can be detected by means of a detection light beam 22. The detection beam 22 is passing through the thermal lens or heat gradient region and then reflected at the interface of the measurement body 16 and the skin of the Fig. 12. Whenever a heat pulse is received from the skin, a local change in refractive index occurs, and this leads to a deflection of the detection beam 22 by the interaction with the material of the measurement body in the region of the thermal lens. In Fig. 1, reference sign 22b corresponds to the non-deflected detection beam 22, whereas reference sign 22a corresponds to the detection beam when it is deflected due to the thermal lens formed in the heat gradient region 20. This deflection can be measured and forms an example of the aforementioned response signal. The degree of the deflection is indicative of the amount of heat received, and hence the degree of absorption of the excitation light 18 in the skin of the finger 12.

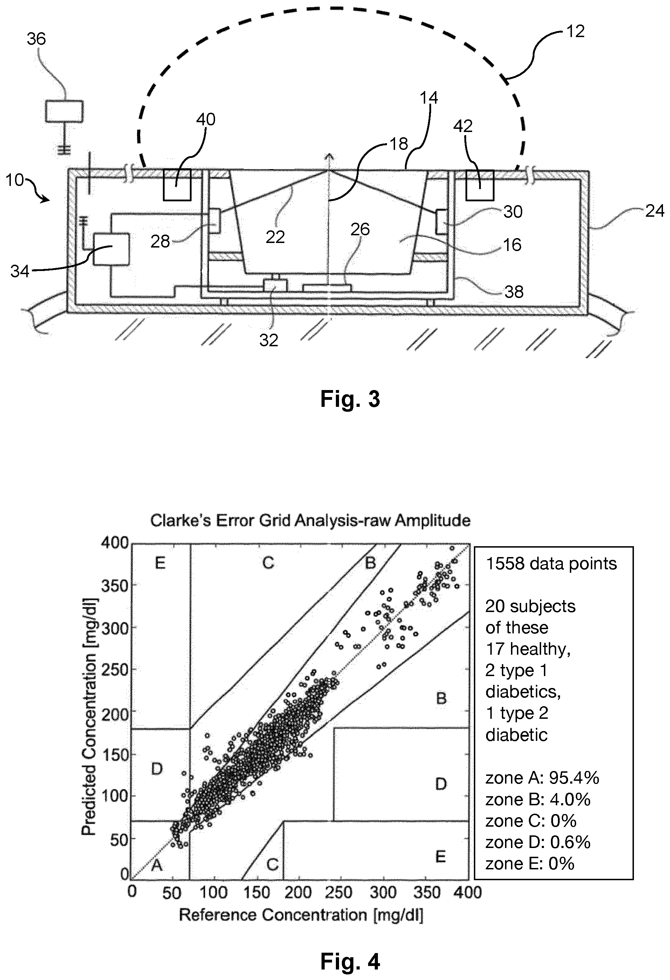

Fig. 3 shows a more detailed sectional view of an apparatus 10 that relies on the measurement principle as illustrated with reference to Fig. 1. The apparatus 10 comprises a housing 24 which includes the measurement body 16, having a top surface (contact surface) 14 on which a body part of the subject/patient such as a finger 12 rests. Within the housing 24, an excitation light source (excitation radiation source) 26 is provided, which generates the excitation light beam 18. In the embodiment shown, the excitation light source 26 comprises an array of quantum cascade lasers (not shown) each having a dedicated wavelength. For example, the array of quantum cascade lasers could include individual quantum cascade laser elements with wavelengths corresponding to the absorption peaks and local minima shown in Fig. 2 (i.e. the glucose-characteristic wavelengths), as well as other wavelengths that can be used for reference measurements (e.g. for determining a response/contact measure, for example one or more characteristic wavelengths of water), or for detecting other substances that could be disturbing to the measurement of the glucose, for example lactate or albumin.

The apparatus 10 further comprises a light source 28, for example a laser, for emitting the detection beam 22, as well as a position-sensitive detector or sensor 30 (e.g. a quadrant photodiode) which allows for detecting the deflection of the detection beam 22. The light source 28 and the position-sensitive detector 30 together form an example of a detection device for detecting the physical response of the measurement body 16 to heat and/or pressure waves received from the fingertip 12. The measurement body 16 in this case is transparent for both, the excitation light beam 18 as well as the detection light beam 22.

In addition, a camera 32 or another imaging device is provided that allows for taking images of the contact surface 14 of the optical medium 16, to thereby record a skin pattern such as a fingerprint of the finger 12 resting on the contact surface 14. This skin pattern can be processed by a control unit 34 such as to determine a response/contact measure and/or to identify and/or authenticate a user via his or her skin pattern (e.g. fingerprint). The camera 32 forms an example of an auxiliary sensor as referred to above that is configured to generate a sensor signal indicative of the degree to which contact between the contact surface and the surface of the skin on the finger 12 permits heat and/or pressure waves generated by absorption of excitation radiation in the tissue to be transferred to the measurement body 16.

The control unit 34 also serves for controlling the light sources 26 and 28 for the excitation light and the detection light, respectively, as well as the sensor 30. The control unit 34 forms an example of a "controller" as referred to above. The control unit 34 may also be in wireless connection with an external data processing device 36 to exchange data. For example, via the wireless connection, user-specific calibration data can be retrieved by the control unit 34 for the user that is identified via the fingerprint. In some examples, the control unit 34 and the external data processing device 36 may together form an example of a "controller" as referred to above. The controller can be comprised by one or more processors, microcontrollers, computers, ASICs, FPGAs, or the like. The controller may be distributed, as indicated in Fig. 3, with various components in data communication with each other, or could be formed by a single control unit, such as the control unit 34, which would be devised for all of the control functionalities described herein. The controller may be generally embodied in hardware, in software, or a combination of both.

As is further seen in Fig. 3, the excitation and detection light sources 26 and 28, as well as the position sensitive detector 30 are all attached to a common carrier structure 38. This means that these components can be preassembled with precision on this structure 38, such that they do not need to be individually adjusted or calibrated when assembling the apparatus 10.

In addition, the apparatus 10 comprises a corneometric device 40 that allows for measuring the water content of the skin. Corneometric devices for measuring the water content in the upper layer of the skin are per se known in the art and need not be described in detail here. For example, known corneometric devices measure the impedance, in particular capacitive impedance of the skin using two interdigital electrodes to which an AC voltage is applied. The corneometric device 40 of Fig. 3 is in contact with the fingertip 12 when the latter rests on the contact surface 14 of the measurement body 16. The corneometric device 40 is an example of the aforementioned "auxiliary sensors", i.e. a sensor that is per se unrelated to the measurement apparatus for measuring the analyte absorption.

The apparatus also comprises a pH-sensor 42 for measuring the pH value of the skin. pH sensors for measuring the pH value on surfaces, including those of the skin are per se known from prior art and need not be described in detail herein. pH sensors for measuring the pH value of the skin are commercially available for medical but also for cosmetic purposes.

Fig. 4 shows results of a Clarke's error grid analysis obtained with an apparatus of the type shown in Fig. 3, illustrating that with the measurement procedure described with reference to Fig. 1 to 3, indeed very reliable blood sugar concentrations can be measured in a purely non-invasive manner. The data shown in Fig. 4 are taken from WO 2017/09782 A1 and do not yet reflect improvements of the present invention. The present invention allows for obtaining results of similar or better quality reproducibly even with smaller and/or cheaper devices (e.g. handheld or wearable devices for everyday use), which may for example comprise less powerful excitation radiation sources than larger stand-alone devices as e.g. employed in a clinical setting.

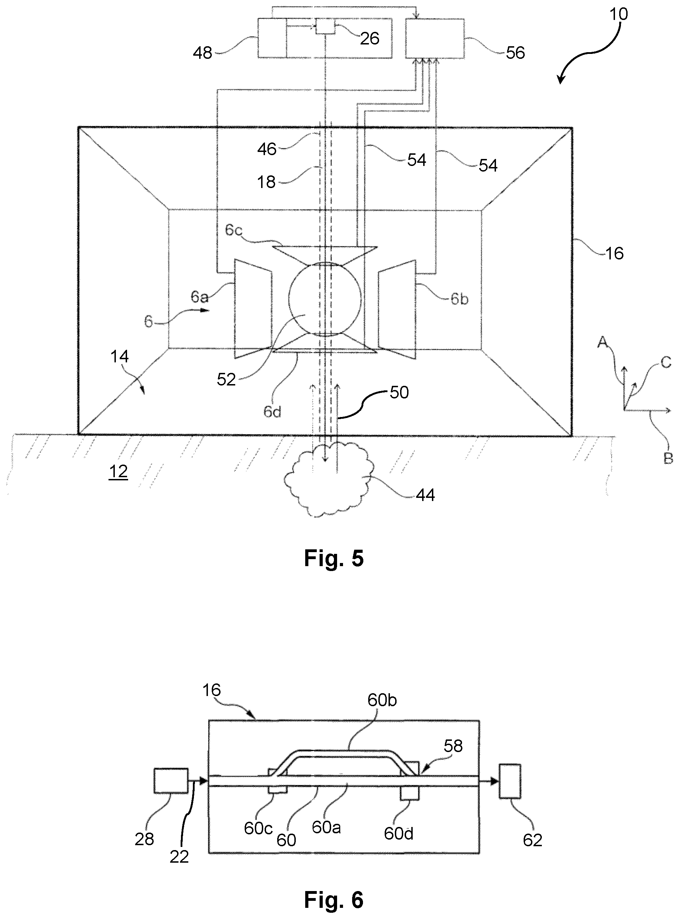

Fig. 5 schematically shows an apparatus 10 which relies on the same general principle involving absorption of excitation radiation for generating heat pulses received by the measurement body 16 from the tissue of the fingertip/body part 12 as that of Fig. 1 and 3, but differs in the physical response exploited and in the way the corresponding response signals are generated. Such an apparatus 10, as well as a large number of variations thereof are described in detail in WO 2019/110597 A2, such that a detailed description may be omitted herein. As before, the apparatus comprises a measurement body 16 having a surface 14 which is brought in contact or coupling with a surface of the skin of a finger 12. Also, a source 26 for an excitation light beam 18 with modulated intensity is provided, which is irradiated into a region 44 underneath the surface of the skin of the finger 12 and absorbed therein. In this embodiment, the excitation light beam 18 runs through a bore 46 indicated by hashed lines through the measurement body 16, such that the measurement body 16 itself need not be transparent for it.

A control unit 48 is provided for modulating the intensity of the excitation light beam 18. This can generally be done in various ways, including a mechanical chopper or an element having a transmissivity or reflectivity that can be electronically controlled. However, in preferred embodiments, the intensity is modulated by modulating the on/off times of the excitation light source 26 as well as the operating current during the on-times thereof. The control unit 48 may for example be integrated into a controller such as the control unit 34 of Fig. 1 at least in part.

A thermal wave caused by the time varying absorption of the intensity modulated excitation beam 18 in the region 44 of the skin of the finger 12 (measurement volume), which is symbolically represented by arrows 50, enters the measuring body 16 where it can be detected in a detection region 52 which has piezoelectric properties. Pressure changes associated with received heat 50 or pressure waves lead to electrical signals that can be recorded with electrodes 6a to 6d, which are connected via conducting leads 54 with an evaluation device 56 for analyzing the tissue (the skin of finger 12). The electrodes 6a to 6d, in some examples together with the evaluation device 56, may form an example of a detection device as referred to above. The evaluation device 56 may be a digital processing device, for example a microcontroller or processor or a computer. In some examples, the evaluation device may be integrated into a controller such as the control unit 34 of Fig. 1 at least in part. In this example, the change in pressure resembles the physical response of the measurement body 16, or other component included therein, to heat received from the tissue of the finger 12 upon absorption of the excitation radiation, which is detected using the piezoelectric properties of the measurement body 16 and the electrodes 6a to 6d, and which leads to electrical signals representing the response signal that is indicative of the degree of absorption of excitation radiation 18.

In alternative variants suggested by the present applicant, as for example disclosed in international application WO 2020/094265 A1, the detection device may comprise an interferometric device allowing for assessing said change in phase of a first part of the detection beam with respect to a second part of the detection beam, wherein only one of the parts of the detection beam passing through a measurement arm is affected by the effects of the heat or pressure wave in the measurement body 16, and generates a response signal indicative of said change in phase. In this example, the physical response of the measurement body 16 (or a component included therein) to heat received from said material 12 upon absorption of said excitation radiation 18 is again a local change in index of refraction, while the response signal is in this case an interferometric signal reflecting a change in the phase of the detection beam due to the local change in refractive index.

This is schematically illustrated in Fig. 6, where a measurement body 16 is shown which is to be brought in contact with a surface of the skin of a body part (such as the finger, not shown in Fig. 6). In this example, the measurement body 16 may be a silicon substrate in which a light guiding structure 58 is provided, which forms an interferometric device 60. The interferometric device 60 forms a Mach-Zehnder interferometer, having a measurement arm 60a and a reference arm 60b. Detection light 22 generated by a detection light source 28 is fed into the light guiding structure 58 and is splitted by a splitter 60c into a portion or a part of the detection beam travelling along the measurement arm 60a and a portion or part travelling along the reference arm 60b, which portions are then united by a combiner 60d. The measurement body 16 is used or arranged such that the reference arm 60a is exposed to heat received from the skin upon absorption of excitation light, but not, or at least to a much lesser extent, the reference arm 60b. Due to received heat, the refractive index in the measurement arm 60a will change, which in turn leads to a phase shift of the detection light 22 travelling along the measurement arm 60a. Since the light travelling along the reference arm 60b is unaffected by the heat received, there will be a change in relative phase of the two portions of light combined by the combiner 60d, which leads to an interference pattern that can be detected using a detector 62. Accordingly, the light source 28, the interferometric device 60 and the detector 62 may together form an example of a detection device as referred to above.

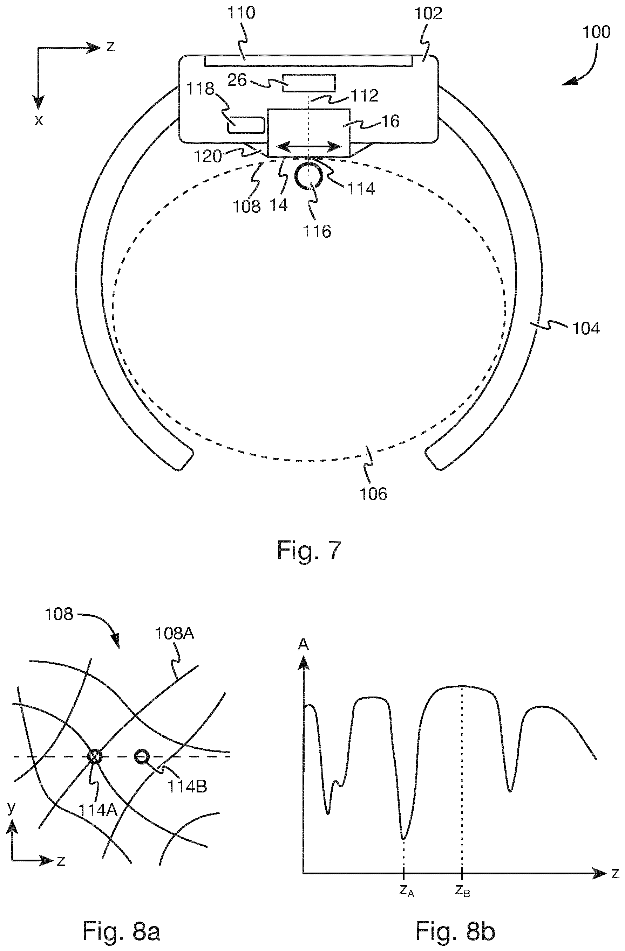

Fig. 7 shows a schematic illustration of a device 100 for detecting an analyte in tissue of a human or animal subject according to an exemplary embodiment of the invention. The device 100 comprises a main housing 102 housing an apparatus for detecting the analyte (e.g. glucose) in the tissue (e.g. in the skin) using a detection method as set forth above. The apparatus for detecting the analyte may rely on any suitable physical response and any suitable way of detecting this physical response, for example as set forth above. The apparatus may in particular be similar to the apparatus of any one of Figs. 3, 5 and 6. As described in more detail below, the apparatus comprises a measurement body 16 with a contact surface 14, an excitation radiation source 26, a detection device (not shown) and a controller (not shown).

In the example of Fig. 7, the device 100 is a wearable device that is configured to be worn (e.g. for extended periods of time or permanently) by the subject. For this, the device 100 comprises a holding portion 104 for the securing the device 100 to a body part 106 of the subject (user/patient), for example to a limb and in particular to an upper arm or a wrist of the subject. The holding portion 104 is configured to be arranged around the body part 106 so as to extend circumferentially around the body part 106 at least in part (as illustrated in Fig. 7a, 7b) or completely. The holding portion 104 may for example be a wristband or a similar structure. The holding portion 104 is configured to secure the device 100 to the body part 106 such that the contact surface 14 of the measurement body 16 faces a surface 108 of the skin on the body part 106, e.g. such that the contact surface 14 is in contact with the skin surface 108. The device 100 further comprises a display 110, which is arranged on or in an upper surface or upper wall of the main housing 102. The display 110 may be configured to display information pertaining to an analyte measurement, in particular a measurement result such as a concentration of the analyte. The display 110 may further be configured to display additional information, e.g. a response/contact measure. In some examples, the device 100 may be a wristwatch, in particular a smartwatch.

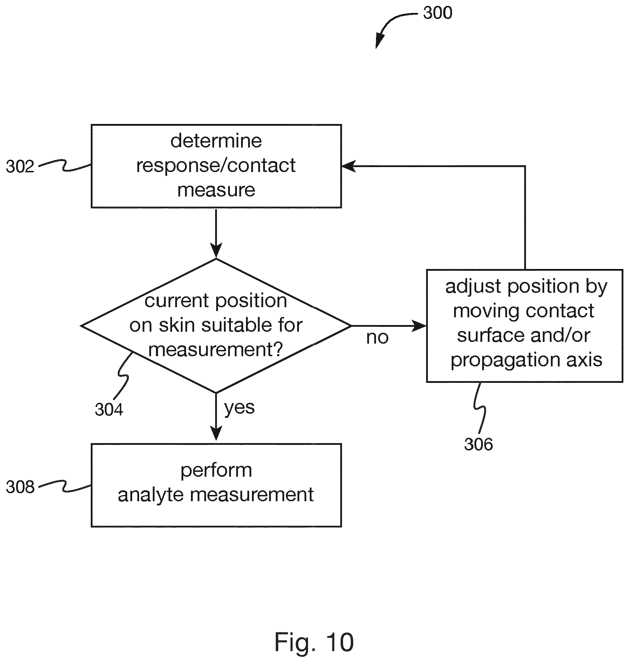

The apparatus for detecting the analyte comprises a measurement body 16 having a contact surface 14 that is suitable to be brought in contact with the skin surface 108 of the subject wearing the device 100 to permit heat and/or pressure waves generated by absorption of excitation radiation in said tissue of the body part 106 to be transferred to the measurement body 16. The apparatus comprises an excitation radiation source 26 for irradiating excitation radiation at a plurality of analyte-characteristic wavelengths into the tissue. The apparatus also comprises a detection device (not shown) for detecting a physical response of the measurement body 16 to heat and/or pressure waves received from the tissue upon absorption of the excitation radiation and for generating a response signal based on the detected physical response. The apparatus further comprises a controller (not shown) for controlling the excitation radiation source 26 and the detection device to perform analyte measurements. The measurement body 16, the excitation radiation source 26, the detection device and/or the controller may for example be embodied as described above with reference to Figs. 3, 5 and 6. In one example, the main housing 102 is similar to or corresponds to the housing 24 of the apparatus 10 of Fig. 3. In some examples, the controller may be integrated into a main control unit of the device 100 at least in part, wherein the main control unit may for example control the display 110 and may be configured to provide smartwatch capabilities as known in the art. The controller of the device 100 may be configured to execute the method 300 for detecting an analyte described below with reference to Fig. 10 at least in part, preferably in its entirety. The excitation radiation source 26 is configured to generate an excitation beam of excitation radiation that propagates through the measurement body 16 and the contact surface 14 along a propagation axis 112. The excitation radiation is irradiated into the tissue in the body part 106 at an irradiation position 114 on the skin surface 108, for example through an irradiation region or spot on the skin surface 108 around the irradiation position 114 that is illuminated by the excitation radiation source 26. In the tissue, the excitation radiation may be absorbed in a measurement volume 116 at and/or below the skin surface 108, thereby generating heat and/or pressure waves that are transferred to the measurement body 16 through a transfer region on the skin surface 108 and through the contact surface 14. The irradiation region and the transfer region may overlap at least in part and may collectively form a measurement region on the skin surface 108.

Fig. 8 shows a schematic illustration of the skin surface 108 of the subject when viewed along the propagation axis 112 (X-axis) in Fig. 7. Rather than forming a flat and uniform surface, the surface 108 of the skin typically has a pronounced microstructure comprising a plurality of microstructural features 108A such as furrows and/or ridges (e.g. friction ridges).

The physical response of the measurement body 16 upon irradiating excitation radiation into the tissue may depend on the position of the irradiation position 114 and of the measurement region associated therewith on the skin surface 108, in particular on their position relative to the microstructural features 108A. The microstructural features 108A may for example affect a transmission efficiency of heat and/or pressure waves from the tissue to the measurement body 16 (e.g. by causing increased attenuation, in particular as a result of air pockets formed between the skin surface 108 and the contact surface 14). Additionally or alternatively, the microstructural features 108A may also affect a transmission efficiency of the excitation radiation into the tissue (e.g. by causing increased or decreased scattering and/or reflection).

This is schematically illustrated in Fig. 8b, which depicts a response/contact measure, namely the amplitude A of a response signal generated by the detection device, as a function of the position z of the irradiation position 114 along the dashed line in Fig. 8a. When the irradiation position 114 is at or close to a microstructural feature 108A (such as the irradiation position 114A at position $z_A$), the amplitude of the response signal may be significantly smaller than when the irradiation position 114 is in a more uniform region between microstructural features 108A (such as the irradiation position 114B at position $z_B$).

When the contact surface 14 is brought in contact with the skin surface 108, the position of the (initial) irradiation position 114 and of the (initial) measurement region associated therewith on the skin surface 108 is typically unknown and cannot be controlled. The initial measurement region may be unsuitable or less suitable for performing an analyte measurement, e.g. due to the presence of a microstructural feature therein. This may affect the reproducibility and accuracy of the measurement. In some cases, a repositioning of the contact surface 14 on the skin surface 108 or vice-versa may be required, for example by removing the contact surface 14 from the skin surface 108 and bringing it in contact with the skin surface 108 again. Since the position of the irradiation position 114 cannot be controlled, however, there is no guarantee that the new measurement region after repositioning is suitable for performing an analyte measurement. The present inventors have found that - depending on the size of the irradiation region and the available optical power - up to 60% of the irradiation positions/measurement regions may be unsuitable for performing an analyte measurement.

The device 100 comprises actuated means 118 that are configured to move the contact surface 14 back and forth transversally with respect to (e.g. perpendicular or substantially perpendicular to) the propagation axis 112 of the excitation radiation, for example by moving the contact surface 14 in a direction that is perpendicular or substantially perpendicular to the propagation axis 112. The actuated means 118 may for example comprise one or more actuators such as for example a piezo element for moving the contact surface 14, e.g. by moving the entire measurement body 16 or a component thereof forming or comprising the contact surface 14. The actuated means may be configured to move the contact surface 14 by at least 500 $\mu$m, preferably at least 1 mm in a direction perpendicular to the propagation axis 112. In some examples, the device 100 may - in addition or instead of the actuated means 118 - comprise deflection means (not shown) configured to move the propagation axis 112 transversally with respect to the contact surface 14, for example as detailed below with reference to Fig. 12.

When the skin surface 108 is in contact with the contact surface 14, the skin surface 108 tends to stick to the contact surface 14 to at least some extent. Accordingly, by moving the contact surface 14, the skin surface 108 may be moved transversally relative to the propagation axis 112. Thereby, the irradiation position 114 on the skin surface 108 can be shifted without having to adjust or move any other optical components. In particular, the propagation axis 112 of the excitation radiation (and optionally of a detection light beam, not shown in Fig. 7) may remain substantially unchanged (for example relative to device 100, e.g. its housing 102). Moving the contact surface 14 instead of e.g. changing the propagation axis 112 of the excitation radiation may allow for a cheaper, simple and more stable optical system as well as for improved measurement accuracy.

In the example of Fig. 7, the measurement body 16 is arranged in a bottom surface or bottom wall of the main housing 102 (i.e. opposite to the display 110) such that the measurement body 16 protrudes from the bottom surface or wall. An interface between the housing 102 (e.g. the bottom surface or wall) and the measurement body 16 may be covered or sealed off by a sealing 120 such as a rubber sealing that allows for moving the contact surface 14 laterally. The sealing 120 may for example cover or seal off exposed portions of the sidewalls of the measurement body 16.

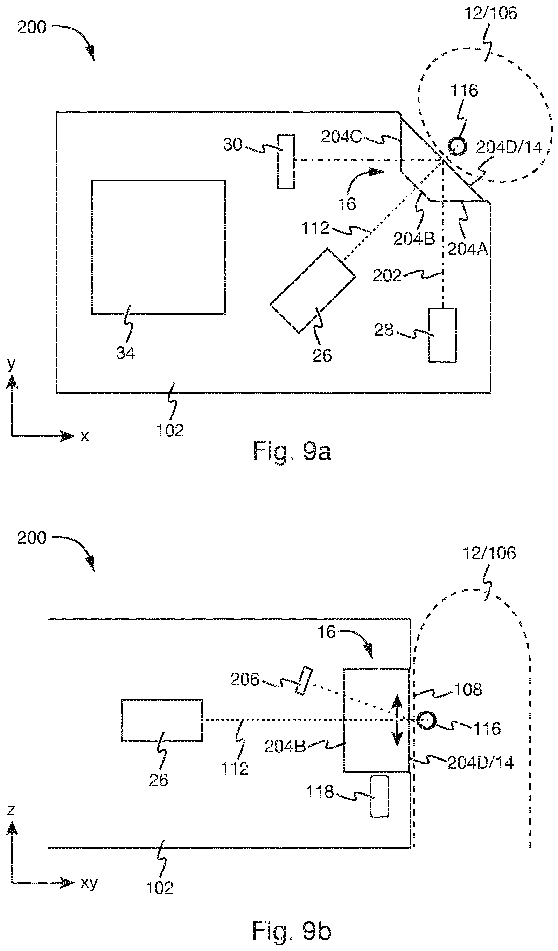

Figs. 9a, 9b show schematic illustrations of a device 200 for detecting an analyte in tissue of a human or animal subject according to another exemplary embodiment of the invention. The device 200 is shown in top view in Fig. 9a (along the Z-axis of Fig. 9b) and in a cross-sectional side view in Fig. 9b (in the plane spanned by the propagation axis 112 and the Z-axis). The device 200 is similar to the device 100 of Fig. 7 and also comprises a housing 102 with an apparatus for detecting the analyte using an analyte measurement as set forth herein. The apparatus comprises a measurement body 16 having a contact surface 14, an excitation radiation source 26, a detection device and a controller/control unit 34. In this example, the apparatus for detecting the analyte is embodied similar to the example of Fig. 3 with the detection device comprising a light source 28 for generating a detection light beam propagating along a propagation axis 202 and a position-sensitive detector 30 for detecting a deflection of the detection light beam. The device 200 may for example be configured for use as a handheld device, wherein an analyte measurement may for example be performed by placing a body part 106 such as a fingertip 12 on the contact surface 14 such that the surface 108 of the skin on the fingertip 12 is in contact with the contact surface 14. The controller 34 may be configured to execute the method 300 and/or the method 400 for detecting an analyte described below with reference to Fig. 10 at least in part, preferably in its entirety.

In the example of Figs. 9a, 9b, the measurement body 16 is a trapezoidal prism that has a first facet 204A, a second facet 204B, a third facet 204C and a fourth facet 204D with the fourth facet 204D (or a part thereof) forming the contact surface 14. Each of the facets 204A-D extends parallel to the direction of view (Z-axis) in Fig. 9a. The first facet 204A forms a (first) input facet through which the detection light beam enters the measurement body 16. The third facet 204C forms an output facet through which the detection light beam leaves the measurement body 16. The first and third facets 204A, 204C are perpendicular (or substantially perpendicular) to the propagation axis 202 of the detection light beam at the respective facet 204A, 204C. The second facet 204B forms a (second) input facet through which the excitation radiation (e.g. the excitation light beam) enters the measurement body 16. The second facet 204B and the fourth facet 204D/contact surface 14 are perpendicular (or substantially perpendicular) to the propagation axis 112 of the excitation radiation at the respective facet 204B, 204D.

In some examples, the propagation axis 112 of the excitation radiation and the propagation axis 202 of the detection light beam lie in the same plane. Some or all of the excitation radiation source 26, the light source 28 and the position-sensitive detector 30 may be arranged in this plane. In some examples, some or all of the excitation radiation source 26, the light source 28 and the position-sensitive detector 30 may be arranged (e.g. attached to or mounted on) a common carrier structure (not shown), wherein the carrier structure preferably is a monolithic carrier structure.

The device 200 comprises actuated means 118 configured to move the measurement body 16 with the contact surface 14 along the Z-axis of Figs. 9a, 9b, i.e. in a direction parallel (or substantially parallel) to the facets 204A-204D of the measurement body 16 and thus perpendicular (or substantially perpendicular) to both the propagation axis 112 of the excitation radiation and the propagation axis 202 of the detection light beam. In some examples, the device 200 may - in addition or instead of the actuated means 118 - comprise deflection means (not shown) configured to move the propagation axis 112 transversally with respect to the contact surface 14, for example as detailed below with reference to Fig. 12.

In some examples, the device 200 may further comprise an auxiliary sensor 206 (not shown in Fig. 9a) that is configured to generate a sensor signal indicative of the degree to which the contact between the contact surface 14 and the skin surface 108 permits heat and/or pressure waves to be transferred from the tissue to the measurement body 16. The auxiliary sensor 206 may for example be an optical sensor that is configured to determine an intensity of light (e.g. of the excitation radiation and/or detection light) that is scattered and/or reflected at the contact surface 14 (e.g. in the vicinity of the propagation axis 112 and/or of a measurement region on the skin surface 108) and/or at the skin surface 108 (e.g. in the vicinity of the propagation axis 112 and/or in a measurement region on the skin surface 108). In some examples, the auxiliary sensor 206 may be an imaging device such as a camera that is configured to take images of a measurement region on the skin surface 108, e.g. similar to the camera 32 of the apparatus 10 of Fig. 3. The intensity of light and/or the images recorded by the auxiliary sensor 206 may for example allow for detecting the presence or absence of microstructural features of the skin surface 108 in the measurement region and/or inferring a response/contact measure for the degree to which heat and/or pressure waves generated by absorption of excitation radiation in the tissue are transferred to the measurement body through the measurement region.

Fig. 10 shows a flow chart of a method 300 for detecting an analyte in tissue of a human or animal subject according to an exemplary embodiment of the invention. The method 300 may be executed using a device for detecting an analyte according to any one of the embodiments disclosed herein, for example the device 100 of Fig. 7 or the device 200 of Figs. 9a, 9b with the latter being used as a non-limiting example for illustrative purposes in the following. Some or all of the steps of method 300 may be executed automatically by the controller of the respective device, for example the controller 34 of the device 200. The method 300 is not limited to the order of execution implied by the flow chart in Fig. 10. As far as technically feasible, the method 300 may be executed in an arbitrary order and parts thereof may be executed simultaneously at least in part, e.g. steps 302 to 306.

The method 300 is executed while the contact surface 14 of the measurement body 16 is in contact with a surface of the skin of the subject, for example the skin surface 108 on a fingertip 12 placed on the contact surface 14 as illustrated in Figs. 9a, 9b. Initially, the skin surface 108 may be placed on the contact surface 14 such that the excitation radiation source 26, when activated, would irradiate excitation radiation into the tissue at an initial irradiation position (associated with an initial measurement region) on the skin surface 108.

In step 302, a response/contact measure is determined for this initial irradiation position/measurement region. The response/contact measure indicates one or both of a degree to which the measurement body 16 exhibits a physical response upon irradiating excitation radiation (at the current, i.e. initial, irradiation position) into the tissue to be absorbed therein and a degree to which said contact permits heat and/or pressure waves generated by absorption of excitation radiation in the tissue to be transferred to said measurement body 16.

The response/contact measure may in particular be determined based on a response signal generated by the detection device 28, 30 and may for example be an amplitude or magnitude of the response signal, e.g. similar as shown in Fig. 8b. The response/contact measure may be determined by performing an analyte measurement, e.g. by irradiating excitation radiation at one or more analyte-characteristic wavelengths at the current irradiation position into the tissue to be absorbed therein using the excitation radiation source 26, detecting the physical response of the measurement body with the detection device 28, 30 and generating the response signal using the detection device 28, 30. Preferably, instead of using said analyte-characteristic wavelengths, the measurement is performed at one or more wavelengths at which the degree of absorption of the excitation radiation is substantially independent of a concentration of the analyte in the tissue, e.g. at one or more characteristic wavelengths of another substance such as water. The response/contact measure can then be determined from the response signal obtained from the measurement, for example by determining a variance or standard deviation of the response signal over time, a peak-to-peak amplitude and/or a Fourier coefficient (e.g. at one or more modulation frequencies used for the measurement) of the response signal. Additionally or alternatively, the response/contact measure may also be determined based on a sensor signal from the auxiliary sensor 206.

In steps 304, 306, the skin surface 108 of the subject and the propagation axis 112 of the excitation radiation are moved transversally with respect to each other based on the response/contact measure determined in step 302. For this, a determination is made in step 304 whether the measurement region or spot associated with the current irradiation position is suitable for performing an analyte measurement. This may for example comprise comparing the response/contact measure to a response/contact threshold. The response/contact threshold may for example be associated with minimum physical response of the measurement body and/or a minimum response signal (e.g. a minimum amplitude of the response signal) required for reliably performing the analyte measurement, for example for reliably determining the concentration of the analyte (e.g. with a measurement uncertainty or error within a desired range). If the response/contact measure meets the response/contact threshold (e.g. the amplitude of the response signal is larger than the minimum amplitude), the method 300 may directly proceed to performing an analyte measurement in step 308. If the response/contact measure does not meet the response/contact threshold (e.g. the amplitude of the response signal is smaller than the minimum amplitude), the method 300 may instead proceed to step 306 to adjust the irradiation position.

In step 306, the skin surface 108 of the subject may for example be moved transversally relative to the propagation axis 112 by moving the contact surface 14 with the actuated means 118 for moving the irradiation position to a different position (adjusted irradiation position) on the skin surface 108. For example, the contact surface 14 may be moved by a predetermined distance in a direction that is perpendicular or substantially perpendicular to the propagation axis 112, e.g. along the Z-axis of Figs. 9a, 9b. The predetermined distance may for example be between 5 µm and 1 mm, in some examples between 10 µm and 200 µm, e.g. 50 µm. Subsequently, the method 300 may return to step 302 to determine a response/contact measure for the adjusted irradiation position. In other examples, the irradiation position may be adjusted by moving the propagation axis 112 transversally relative to the skin surface 108 (either in addition or instead of moving the skin surface 108).

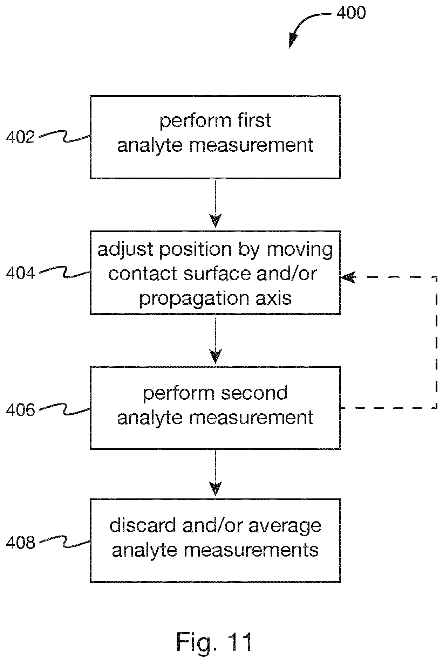

This procedure may be repeated until the response/contact measure meets the response/contact threshold or for optimizing the response/contact measure, for example until an optimized irradiation position associated with (local) optimum (e.g. maximum) of the response/contact measure is found. In other examples, method 300 may not comprise step 304 but the irradiation position may always be adjusted in step 306 prior to performing the analyte measurement in step 308, for example to find an optimized irradiation position or as in the method 400 of Fig. 11 described below.

In step 308, an analyte measurement is performed at the adjusted measurement region determined in steps 302 to 306, for example at the initial measurement region in case the response/contact measure for the initial irradiation position/measurement region meets the response/contact threshold or at a shifted measurement region in case the response/contact measure for the initial irradiation position/measurement region does not meet the response/contact threshold. For performing the analyte measurement, excitation radiation may be irradiated into the tissue at one or more analyte-characteristic wavelengths at the adjusted irradiation position determined in steps 302-306 using the excitation radiation source 26. The physical response of the measurement body or of a component included therein may be detected with the detection device 28, 30 and a response signal indicative of the degree of absorption of said excitation radiation may be generated using the detection device 28, 30.

In some examples, the method 300 may comprise performing an active feedback control for continuously adjusting the irradiation position/measurement region based on the response/contact measure. For this, steps 302 to 306 may for example be executed repeatedly, e.g. at predetermined time intervals and/or after a predetermined number of analyte measurements.

In some embodiments, moving the skin surface 108 and the propagation axis 112 relative to each other may not be based on a response/contact measure as in the example of Fig. 10 but the skin surface 108 and the propagation axis 112 may for example be moved between subsequent analyte measurements independent of the response/contact measure, e.g. to sample or scan different irradiation positions. An example for such a method 400 is depicted as a flow chart in Fig. 11 and described in the following using the device 200 of Figs. 9a, 9b as a non-limiting example for illustrative purposes.

The method 400 is executed while the contact surface 14 of the measurement body 16 is in contact with a surface of the skin of the subject, for example the skin surface 108 on a fingertip 12 placed on the contact surface 14 as illustrated in Figs. 9a, 9b. Initially, the skin surface 108 may be placed on the contact surface 14 such that the excitation radiation source 26, when activated, would irradiate excitation radiation into the tissue at an initial irradiation position (associated with an initial measurement region) on the skin surface 108.

In step 402, a first analyte measurement is performed at the initial irradiation position, e.g. similar to step 308 of method 300. In some examples, the first analyte measurement may amount to (e.g. comprise) the determination of a first response/contact measure (associated with the initial irradiation position). The response/contact measure may for example be a parameter or quantity that is any way determined as part of an analyte measurement, for example the amplitude of the response signal at one or more analyte-characteristic wavelengths and/or the difference in amplitude of the response signal between two analyte-characteristic wavelengths. The response/contact measure may for example be used for deciding whether or not to discard the first analyte measurement and/or for weighting the first analyte measurement.

In step 404, the irradiation position on the skin surface 108 is adjusted by moving the skin surface 108 and the propagation axis 112 transversally with respect to each other, e.g. by moving the contact surface 14 using the actuated means 118 and/or by moving the propagation axis 112 using suitable deflection means. In step 406, a second analyte measurement is performed at the adjusted irradiation position, which may be performed in the same way as the first analyte measurement. The second analyte measurement may amount to (e.g. comprise) the determination of a second response/contact measure (associated with the adjusted irradiation position).

In some examples, the skin surface 108 and the propagation axis 112 may already have been moved with respect to each other (using the corresponding means) prior to step 402, e.g. prior to execution of the method 400 or as part of the method 400. The initial irradiation position thus does not necessarily correspond to the irradiation position when the skin surface 108 is first placed on the contact surface 14 but may in itself also be an adjusted irradiation position.

In some examples, steps 404 and 406 may be executed repeatedly as indicated by the dashed arrow in Fig. 11, e.g. to perform analyte measurements at different irradiation positions. For example, the skin surface 108 and the propagation axis 112 may be moved transversally with respect to each other in step 404 so as to sample or scan a plurality of irradiation positions on the skin surface 108, e.g. in a regular or irregular one-dimensional or two-dimensional pattern.

In some examples, the method may further comprise discarding and/or averaging analyte measurements in step 408. For example, analyte measurements may be discarded if the respective response/contact measure does not meet a corresponding threshold. The analyte measurements that were not discarded (i.e. for which the respective response/contact meets the threshold) may subsequently be averaged. In another example, all analyte measurements are averaged (independently of the response/contact measure). In some examples, the averaging may comprise weighting the analyte measurements based on the respective response/contact measure, either in addition to or instead of the discarding to analyte measurements.

Fig. 12 shows a schematic illustration of a device 500 for detecting an analyte in tissue of a human or animal subject according to another exemplary embodiment of the invention. The device 500 is similar to the devices 100 and 200 described above (with corresponding elements being denoted by the same reference signs) but employs an acoustic detection (instead of an optical detection) and comprises deflection means 502 for moving the propagation axis 112 relative to the contact surface 14 (instead of the actuated means 118).

In the example of Fig. 12, the deflection means 502 are embodied as a tiltable mirror that is configured to be tilted (e.g. by an actuated element such as a piezo element) for adjusting the angle by which the excitation irradiation is deflected from the mirror. Thereby, the propagation axis 112 may be moved (tilted) transversally with respect to the contact surface 14 of the measurement body 16. The contact surface 14 and/or the measurement body 16 may be fixed within the housing 102 in some examples, i.e. may not be moveable relative to the housing 102. In some examples, the device 500 may - in addition or instead of the deflection means 502 - comprise actuated means (not shown) configured to move the contact surface 14 transversally with respect to the propagation axis 112, for example similar to the device 100, 200 of Figs. 7, 9a and 9b.

For generating a response signal indicative of the degree of absorption of excitation radiation in the tissue, the measurement body 16 comprises a recess 16A in the contact surface 14. When the contact surface 14 is brought in contact with the skin surface 108, the recess 16A may form a void or cavity within the measurement body 16 to which heat and/or pressure waves generated by absorption of excitation radiation in the tissue may be transferred (e.g. by excitation of and/or conversion to sound waves within the cavity/recess 16A). These sound waves within the cavity/recess 16A may constitute the physical response of the measurement body 16. In some examples, the recess 16A may be covered, e.g. by the contact surface 14 or another component of the measurement body 16, and/or may be embodied as a void or cavity within the measurement body 16 (e.g. below the contact surface 14).

To detect the physical response, the device 500 comprises a microphone 304 (e.g. as an example or a part of a detection device). The microphone 304 may be arranged within the recess (e.g. on a wall of the recess 16A) or in (e.g. embedded in) a wall of the recess as illustrated in Fig. 12. In other examples, the microphone 304 may be arranged in other parts of the measurement body 16 or the device 300 but may be acoustically coupled to the recess/cavity 16A. The microphone 304 may be configured to detect sound waves in the cavity/recess 16A and to generate a response signal (e.g. an electrical response signal) based on the detected sound waves.

While the present invention has been described in terms of specific embodiments, it is understood that variations and modifications will occur to those in the art, all of which are intended as aspects of the present invention. Accordingly, only such limitations as appear in the claims should be placed on the invention.

**Claims**

1. A device (100, 200, 500) for detecting an analyte in tissue of a human or animal subject, the device (100, 200, 500) comprising:

   a measurement body (16) having a contact surface (14) suitable to be brought in contact with a skin surface (108) of the subject, said contact permitting heat and/or pressure waves generated by absorption of excitation radiation in the tissue to be transferred to said measurement body (16);
   an excitation radiation source (26) configured for irradiating excitation radiation at a plurality of wavelengths into the tissue to be absorbed by the analyte contained therein;
   a detection device (28, 30, 504) for detecting a physical response of the measurement body (16) or of a component included therein to a heat and/or pressure wave received from the tissue upon absorption of the excitation radiation and for generating a response signal based on said detected physical response, said response signal being indicative of a degree of absorption of excitation radiation; and
   a controller (34) to control the excitation radiation source (26) to irradiate excitation radiation into the tissue to be absorbed by the analyte contained therein and to control the detection device (28, 30, 504) to detect said physical response and to generate a response signal indicative of the degree of absorption of said excitation radiation,
   wherein the device (100, 200, 500) further comprises means (118, 502) configured to move said contact surface (14) and a propagation axis (112) of the excitation radiation irradiated into the tissue transversally with respect to each other while the contact surface (14) is in contact with the skin surface (108) of the subject to adjust an irradiation position (114) on the skin surface (108) at which said excitation radiation is to be irradiated into the tissue,
**characterized in that** said controller (34) is configured to:

   determine a response/contact measure (A), said response/contact measure (A) indicating one or both of a degree to which said measurement body (16) exhibits a physical response upon irradiating excitation radiation into the tissue to be absorbed therein and a degree to which said contact permits heat and/or pressure waves generated by absorption of excitation radiation in the tissue to be transferred to said measurement body (16); and
   control said means (118, 502) to move the contact surface (14) and the propagation axis (112) of the excitation radiation transversally with respect to each other based on said response/contact measure (A).

2. The device (100, 200, 500) of claim 1, wherein said analyte is glucose, in particular wherein said tissue is the skin of the subject and said analyte is glucose present in an interstitial fluid thereof.

3. The device (100, 200, 500) of claim 1 or 2, wherein the controller (34) is configured to determine the response/contact measure (A) based on a response signal generated by said detection device (28, 30, 504), in particular wherein the controller (34) is configured to determine the response/contact measure (A) by:

   controlling the excitation radiation source (26) to irradiate excitation radiation into the tissue to be absorbed therein at one or more wavelengths at which the degree of absorption of said excitation radiation is substantially independent of a concentration of the analyte in the tissue; and
   controlling the detection device (28, 30, 504) to detect said physical response and to generate a response signal indicative of the degree of absorption of said excitation radiation.

4. The device (100, 200, 500) of any one of claims 1 to 3, further comprising an auxiliary sensor (206) configured to generate a sensor signal that is indicative of the degree to which said contact permits heat and/or pressure waves generated by absorption of excitation radiation in the tissue to be transferred to said measurement body (16), wherein the controller (34) is configured to determine the response/contact measure (A) based on the sensor signal generated by the auxiliary sensor (206).

5. The device (100, 200) of any one of the preceding claims, wherein said means (118) configured to move the contact surface (14) and the propagation axis (112) of the excitation radiation transversally with respect to each other are or comprise actuated means (118) configured to move the contact surface (14) transversally with respect to the propagation axis (112) of the excitation radiation irradiated into the tissue while the contact surface (14) is in contact with the skin surface (108) of the subject, in particular wherein said actuated means (118) comprise:

one or more actuators, said one or more actuators comprising one or more of a piezo element, an electric actuator, a magnetic actuator and a fluid pump; and/or
one or more deformable members configured to be deformed, extended and/or retracted, said one or more deformable members comprising one or more of an inflatable body and/or a pneumatically and/or hydraulically extendable member.

6. The device (100, 200) of claim 5, wherein said actuated means (118) are configured to move said contact surface (14) by moving the entire measurement body (16) or a component thereof.

7. The device (100, 200) of claim 5 or 6, wherein said detection device (28, 30) comprises a light source for generating a detection light beam (22) travelling through at least a portion of said measurement body (16) or of a component included therein and said actuated means (118) are configured to move said contact surface (14) in a direction that is perpendicular or substantially perpendicular to both the propagation axis (112) of said excitation radiation irradiated into the tissue and a propagation axis (202) of said detection light beam (22), wherein preferably said measurement body (16) comprises a prism, in particular a trapezoidal prism, having a first input facet (204A) that is perpendicular or substantially perpendicular to the propagation axis (112) of said excitation radiation and a second input facet (204B) that is perpendicular or substantially perpendicular to the propagation axis (202) of said detection light beam (22), wherein said actuated means (118) are configured to move said prism along with said contact surface (14) in a direction parallel or substantially parallel to the first and second input facets (204A, 204B).

8. The device (100, 200) of any one of claims 5 to 7, wherein said actuated means (118) are further configured to move said contact surface (14) longitudinally with respect to the propagation axis (112) of said excitation radiation for temporarily increasing a contact pressure between the measurement body (16) and the skin surface (108) of the subject.

9. A method (300, 400) for detecting an analyte in tissue of a human or animal subject using a device (100, 200, 500) according to any one of the preceding claims, wherein the contact surface (14) of the measurement body (16) is in contact with a skin surface (108) of the subject and the method (300, 400) comprises:

determining a response/contact measure (A), said response/contact measure (A) indicating one or both of a degree to which said measurement body (16) exhibits a physical response upon irradiating excitation radiation into the tissue to be absorbed therein and a degree to which said contact permits heat and/or pressure waves generated by absorption of excitation radiation in the tissue to be transferred to said measurement body (16);
moving the skin surface (108) of the subject and the propagation axis (112) of the excitation radiation transversally with respect to each other by moving one or both of the contact surface (14) and the propagation axis (112) of the excitation radiation using said means (118, 502) with the contact surface (14) being in contact with the skin surface (108) of the subject to adjust an irradiation position (114) on the skin surface (108) at which said excitation radiation is to be irradiated into the tissue, wherein the skin surface (108) of the subject and the propagation axis (112) of the excitation radiation are moved transversally with respect to each other based on said response/contact measure (A); and

performing an analyte measurement by irradiating excitation radiation at the adjusted irradiation position (114) into the tissue to be absorbed by the analyte contained therein using the excitation radiation source (26), detecting the physical response of the measurement body (16) or a component included therein with the detection device (28, 30, 504) and generating a response signal indicative of the degree of absorption of said excitation radiation using the detection device (28, 30, 504).

10. The method (300, 400) of claim 9, wherein:

the method (300, 400) further comprises comparing the response/contact measure (A) with a response/contact threshold, wherein the skin surface (108) of the subject and the propagation axis (112) of the excitation radiation are moved transversally with respect to each other in response to determining that the response/contact measure (A) does not meet the response/contact threshold; and/or
the method (300, 400) further comprises optimizing the response/contact measure (A) by moving the skin surface (108) of the subject and the propagation axis (112) of the excitation radiation transversally with respect to each other; and/or

the response/contact measure (A) is determined repeatedly and the method (300, 400) further comprises performing an active feedback control based on said response/contact measure (A) to adjust said irradiation position (114) by moving the skin surface (108) of the subject and the propagation axis (112) of the excitation radiation transversally with respect to each other using said means (118, 502).

11. The method (300, 400) of claim 9 or 10, wherein determining the response/contact measure (A) comprises:

controlling the excitation radiation source (26) to irradiate excitation radiation at a current irradiation position (114) into the tissue to be absorbed therein, in particular at one or more wavelengths at which the degree of absorption of said excitation radiation is substantially independent of a concentration of the analyte in the tissue; controlling the detection device (28, 30, 504) to detect said physical response and to generate a response signal indicative of the degree of absorption of said excitation radiation; and determining the response/contact measure (A) based on said response signal.

12. The method (300, 400) of any one of claims 9 to 11, further comprising performing another analyte measurement at an initial irradiation position (114) prior to moving the skin surface (108) of the subject and the propagation axis (112) of the excitation radiation transversally with respect to each other, in particular wherein the method (300, 400) comprises repeatedly performing analyte measurements and moving the skin surface (108) of the subject and the propagation axis (112) of the excitation radiation transversally with respect to each other between said analyte measurements to sample or scan a plurality of irradiation positions (114).

13. The device (100, 200, 500) of any one of claims 1 to 8, wherein the controller (34) is configured to execute a method (300, 400) according to any one of claims 9 to 12.

**Patentansprüche**

1. Vorrichtung (100, 200, 500) zum Detektieren eines Analyten in Gewebe eines menschlichen oder tierischen Subjekts, wobei die Vorrichtung (100, 200, 500) Folgendes umfasst:

einen Messkörper (16) mit einer Kontaktfläche (14), die dazu geeignet ist, mit einer Hautoberfläche (108) des Subjekts in Kontakt gebracht zu werden, wobei der Kontakt ermöglicht, dass Wärme- und/oder Druckwellen, die durch Absorption von Anregungsstrahlung in dem Gewebe erzeugt werden, auf den Messkörper (16) übertragen werden; eine Anregungsstrahlungsquelle (26), die dazu konfiguriert ist, Anregungsstrahlung bei einer Vielzahl von Wellenlängen in das Gewebe einzustrahlen, um von dem darin enthaltenen Analyten absorbiert zu werden; eine Detektionsvorrichtung (28, 30, 504) zum Detektieren einer physikalischen Reaktion des Messkörpers (16) oder einer darin enthaltenen Komponente auf eine Wärme- und/oder Druckwelle, die von dem Gewebe bei Absorption der Anregungsstrahlung empfangen wird, und zum Erzeugen eines Reaktionssignals basierend auf der detektierten physikalischen Reaktion, wobei das Reaktionssignal einen Grad der Absorption von Anregungsstrahlung anzeigt; und eine Steuerung (34), um die Anregungsstrahlungsquelle (26) zu steuern, Anregungsstrahlung in das Gewebe einzustrahlen, um von dem darin enthaltenen Analyten absorbiert zu werden, und um die Detektionsvorrichtung (28, 30, 504) zu steuern, die physikalische Reaktion zu detektieren und ein Reaktionssignal zu erzeugen, das den Grad der Absorption der Anregungsstrahlung anzeigt, wobei die Vorrichtung (100, 200, 500) ferner Mittel (118, 502) umfasst, die dazu konfiguriert sind, die Kontaktfläche (14) und eine Propagationsachse (112) der in das Gewebe eingestrahlten Anregungsstrahlung transversal zueinander zu bewegen, während die Kontaktfläche (14) mit der Hautoberfläche (108) des Subjekts in Kontakt steht, um eine Bestrahlungsposition (114) auf der Hautoberfläche (108) anzupassen, an der die Anregungsstrahlung in das Gewebe eingestrahlt werden soll, **dadurch gekennzeichnet, dass** die Steuerung (34) konfiguriert ist zum:

Bestimmen eines Reaktions-/Kontaktmaßes (A), wobei das Reaktions-/Kontaktmaß (A) eines oder beides von einem Grad, in dem der Messkörper (16) eine physikalische Reaktion beim Einstrahlen von Anregungsstrahlung in das Gewebe zur Absorption darin zeigt, und einem Grad, in dem der Kontakt ermöglicht, dass Wärme- und/oder Druckwellen, die durch Absorption von Anregungsstrahlung in dem Gewebe erzeugt werden, auf den Messkörper (16) übertragen werden, anzeigt; und

Steuern der Mittel (118, 502), um die Kontaktfläche (14) und die Propagationsachse (112) der Anregungsstrahlung transversal zueinander basierend auf dem Reaktions-/Kontaktmaß (A) zu bewegen.

2. Vorrichtung (100, 200, 500) nach Anspruch 1, wobei der Analyt Glukose ist, insbesondere wobei das Gewebe die Haut des Subjekts ist und der Analyt Glukose ist, die in einer interstitiellen Flüssigkeit davon vorhanden ist.

3. Vorrichtung (100, 200, 500) nach Anspruch 1 oder 2, wobei die Steuerung (34) konfiguriert ist, das Reaktions-/Kontaktmaß (A) basierend auf einem Reaktionssignal zu bestimmen, das von der Detektionsvorrichtung (28, 30, 504) erzeugt wird, insbesondere wobei die Steuerung (34) konfiguriert ist, das Reaktions-/Kontaktmaß (A) zu bestimmen durch:

Steuern der Anregungsstrahlungsquelle (26), um Anregungsstrahlung in das Gewebe zur Absorption darin bei einer oder mehreren Wellenlängen einzustrahlen, bei denen der Grad der Absorption der Anregungsstrahlung im Wesentlichen unabhängig von einer Konzentration des Analyten in dem Gewebe ist; und Steuern der Detektionsvorrichtung (28, 30, 504), um die physikalische Reaktion zu detektieren und ein Reaktionssignal zu erzeugen, das den Grad der Absorption der Anregungsstrahlung anzeigt.

4. Vorrichtung (100, 200, 500) nach einem der Ansprüche 1 bis 3, ferner umfassend einen Hilfssensor (206), der konfiguriert ist, ein Sensorsignal zu erzeugen, das den Grad anzeigt, in dem der Kontakt ermöglicht, dass Wärme- und/oder Druckwellen, die durch Absorption von Anregungsstrahlung in dem Gewebe erzeugt werden, auf den Messkörper (16) übertragen werden, wobei die Steuerung (34) konfiguriert ist, das Reaktions-/Kontaktmaß (A) basierend auf dem Sensorsignal zu bestimmen, das von dem Hilfssensor (206) erzeugt wird.

5. Vorrichtung (100, 200) nach einem der vorhergehenden Ansprüche, wobei die Mittel (118), die dazu konfiguriert sind, die Kontaktfläche (14) und die Propagationsachse (112) der Anregungsstrahlung transversal zueinander zu bewegen, aktuierte Mittel (118) sind oder umfassen, die dazu konfiguriert sind, die Kontaktfläche (14) transversal zu der Propagationsachse (112) der in das Gewebe eingestrahlten Anregungsstrahlung zu bewegen, während die Kontaktfläche (14) mit der Hautoberfläche (108) des Subjekts in Kontakt steht, insbesondere wobei die aktuierten Mittel (118) Folgendes umfassen:

einen oder mehrere Aktuatoren, wobei der eine oder die mehreren Aktuatoren eines oder mehrere von einem Piezoelement, einem elektrischen Aktuator, einem magnetischen Aktuator und einer Fluidpumpe umfassen; und/oder ein oder mehrere verformbare Elemente, die dazu konfiguriert sind, verformt, ausgefahren und/oder eingefahren zu werden, wobei das eine oder die mehreren verformbaren Elemente eines oder mehrere von einem aufblasbaren Körper und/oder einem pneumatisch und/oder hydraulisch ausfahrbaren Element umfassen.

6. Vorrichtung (100, 200) nach Anspruch 5, wobei die aktuierten Mittel (118) dazu konfiguriert sind, die Kontaktfläche (14) durch Bewegen des gesamten Messkörpers (16) oder einer Komponente davon zu bewegen.

7. Vorrichtung (100, 200) nach Anspruch 5 oder 6, wobei die Detektionsvorrichtung (28, 30) eine Lichtquelle zum Erzeugen eines Detektionslichtstrahls (22) umfasst, der sich durch mindestens einen Abschnitt des Messkörpers (16) oder einer darin enthaltenen Komponente bewegt, und die aktuierten Mittel (118) dazu konfiguriert sind, die Kontaktfläche (14) in eine Richtung zu bewegen, die senkrecht oder im Wesentlichen senkrecht zu sowohl der Propagationsachse (112) der in das Gewebe eingestrahlten Anregungsstrahlung als auch einer Propagationsachse (202) des Detektionslichtstrahls (22) ist, wobei der Messkörper (16) vorzugsweise ein Prisma, insbesondere ein trapezförmiges Prisma, mit einer ersten Eingangsfacette (204A), die senkrecht oder im Wesentlichen senkrecht zu der Propagationsachse (112) der Anregungsstrahlung ist, und einer zweiten Eingangsfacette (204B), die senkrecht oder im Wesentlichen senkrecht zu der Propagationsachse (202) des Detektionslichtstrahls (22) ist, umfasst, wobei die aktuierten Mittel (118) dazu konfiguriert sind, das Prisma zusammen mit der Kontaktfläche (14) in eine Richtung parallel oder im Wesentlichen parallel zu der ersten und zweiten Eingangsfacette (204A, 204B) zu bewegen.

8. Vorrichtung (100, 200) nach einem der Ansprüche 5 bis 7, wobei die aktuierten Mittel (118) ferner dazu konfiguriert sind, die Kontaktfläche (14) longitudinal zu der Propagationsachse (112) der Anregungsstrahlung zu bewegen, um temporär einen Kontaktdruck zwischen dem Messkörper (16) und der Hautoberfläche (108) des Subjekts zu erhöhen.

9. Verfahren (300, 400) zum Detektieren eines Analyten in Gewebe eines menschlichen oder tierischen Subjekts unter Verwendung einer Vorrichtung (100, 200, 500) nach einem der vorhergehenden Ansprüche, wobei die Kontaktfläche (14) des Messkörpers (16) mit einer Hautoberfläche (108) des Subjekts in Kontakt steht und das Verfahren (300, 400) Folgendes umfasst:

Bestimmen eines Reaktions-/Kontaktmaßes (A), wobei das Reaktions-/Kontaktmaß (A) eines oder beides von einem Grad, in dem der Messkörper (16) eine physikalische Reaktion beim Einstrahlen von Anregungsstrahlung in das Gewebe zur Absorption darin zeigt, und einem Grad, in dem der Kontakt ermöglicht, dass Wärme- und/oder Druckwellen, die durch Absorption von Anregungsstrahlung in dem Gewebe erzeugt werden, auf den Messkörper (16) übertragen werden, anzeigt;

Bewegen der Hautoberfläche (108) des Subjekts und der Propagationsachse (112) der Anregungsstrahlung transversal zueinander durch Bewegen einer oder beider von der Kontaktfläche (14) und der Propagationsachse (112) der Anregungsstrahlung unter Verwendung der Mittel (118, 502), wobei die Kontaktfläche (14) mit der Hautoberfläche (108) des Subjekts in Kontakt steht, um eine Bestrahlungsposition (114) auf der Hautoberfläche (108) anzupassen, an der die Anregungsstrahlung in das Gewebe eingestrahlt werden soll, wobei die Hautoberfläche (108) des Subjekts und die Propagationsachse (112) der Anregungsstrahlung transversal zueinander basierend auf dem Reaktions-/Kontaktmaß (A) bewegt werden; und

Durchführen einer Analytmessung durch Einstrahlen von Anregungsstrahlung an der angepassten Bestrahlungsposition (114) in das Gewebe, um von dem darin enthaltenen Analyten absorbiert zu werden, unter Verwendung der Anregungsstrahlungsquelle (26), Detektieren der physikalischen Reaktion des Messkörpers (16) oder einer darin enthaltenen Komponente mit der Detektionsvorrichtung (28, 30, 504) und Erzeugen eines Reaktionssignals, das den Grad der Absorption der Anregungsstrahlung anzeigt, unter Verwendung der Detektionsvorrichtung (28, 30, 504).

10. Verfahren (300, 400) nach Anspruch 9, wobei:

das Verfahren (300, 400) ferner das Vergleichen des Reaktions-/Kontaktmaßes (A) mit einem Reaktions-/Kontaktschwellenwert umfasst, wobei die Hautoberfläche (108) des Subjekts und die Propagationsachse (112) der Anregungsstrahlung transversal zueinander als Reaktion auf das Bestimmen, dass das Reaktions-/Kontaktmaß (A) den Reaktions-/Kontaktschwellenwert nicht erfüllt, bewegt werden; und/oder das Verfahren (300, 400) ferner das Optimieren des Reaktions-/Kontaktmaßes (A) durch Bewegen der Hautoberfläche (108) des Subjekts und der Propagationsachse (112) der Anregungsstrahlung transversal zueinander umfasst; und/oder

das Reaktions-/Kontaktmaß (A) wiederholt bestimmt wird und das Verfahren (300, 400) ferner das Durchführen einer aktiven Rückkopplungssteuerung basierend auf dem Reaktions-/Kontaktmaß (A) umfasst, um die Bestrahlungsposition (114) durch Bewegen der Hautoberfläche (108) des Subjekts und der Propagationsachse (112) der Anregungsstrahlung transversal zueinander unter Verwendung der Mittel (118, 502) anzupassen.

11. Verfahren (300, 400) nach Anspruch 9 oder 10, wobei das Bestimmen des Reaktions-/Kontaktmaßes (A) umfasst:

Steuern der Anregungsstrahlungsquelle (26), um Anregungsstrahlung an einer aktuellen Bestrahlungsposition (114) in das Gewebe zur Absorption darin einzustrahlen, insbesondere bei einer oder mehreren Wellenlängen, bei denen der Grad der Absorption der Anregungsstrahlung im Wesentlichen unabhängig von einer Konzentration des Analyten in dem Gewebe ist; Steuern der Detektionsvorrichtung (28, 30, 504), um die physikalische Reaktion zu detektieren und ein Reaktionssignal zu erzeugen, das den Grad der Absorption der Anregungsstrahlung anzeigt; und Bestimmen des Reaktions-/Kontaktmaßes (A) basierend auf dem Reaktionssignal.

12. Verfahren (300, 400) nach einem der Ansprüche 9 bis 11, ferner umfassend das Durchführen einer weiteren Analytmessung an einer anfänglichen Bestrahlungsposition (114) vor dem Bewegen der Hautoberfläche (108) des Subjekts und der Propagationsachse (112) der Anregungsstrahlung transversal zueinander, insbesondere wobei das Verfahren (300, 400) das wiederholte Durchführen von Analytmessungen und das Bewegen der Hautoberfläche (108) des Subjekts und der Propagationsachse (112) der Anregungsstrahlung transversal zueinander zwischen den Analytmessungen umfasst, um eine Vielzahl von Bestrahlungspositionen (114) abzutasten oder abzuscannen.

13. Vorrichtung (100, 200, 500) nach einem der Ansprüche 1 bis 8, wobei die Steuerung (34) konfiguriert ist, ein Verfahren (300, 400) nach einem der Ansprüche 9 bis 12 auszuführen.

**Revendications**

1. Dispositif (100, 200, 500) de détection d'un analyte dans un tissu d'un sujet humain ou animal, le dispositif (100, 200, 500) comprenant :

   un corps de mesure (16) ayant une surface de contact (14) adaptée pour être amenée en contact avec une surface de peau (108) du sujet, ledit contact permettant aux ondes de chaleur et/ou de pression générées par l'absorption du rayonnement d'excitation dans le tissu d'être transférées vers ledit corps de mesure (16) ;
   une source de rayonnement d'excitation (26) configurée pour irradier un rayonnement d'excitation à une pluralité de longueurs d'onde vers le tissu afin qu'il soit absorbé par l'analyte contenu dans celui-ci ;
   un dispositif de détection (28, 30, 504) destiné à détecter une réponse physique du corps de mesure (16) ou d'un composant inclus dans celui-ci à une onde de chaleur et/ou de pression reçue de la part du tissu lors de l'absorption du rayonnement d'excitation, et à générer un signal de réponse sur la base de ladite réponse physique détectée, ledit signal de réponse indiquant un degré d'absorption du rayonnement d'excitation ; et
   un dispositif de commande (34) destiné à contrôler la source de rayonnement d'excitation (26) afin d'irradier le rayonnement d'excitation vers le tissu de sorte qu'il soit absorbé par l'analyte contenu dans celui-ci, et à contrôler le dispositif de détection (28, 30, 504) afin de détecter ladite réponse physique et à générer un signal de réponse qui indique le degré d'absorption dudit rayonnement d'excitation,
   dans lequel le dispositif (100, 200, 500) comprend en outre un moyen (118, 502) configuré pour déplacer ladite surface de contact (14) et un axe de propagation (112) du rayonnement d'excitation irradié vers le tissu de manière transversale l'un par rapport à l'autre pendant que la surface de contact (14) est en contact avec la surface de peau (108) du sujet afin de régler une position d'irradiation (114) sur la surface de peau (108) au niveau de laquelle ledit rayonnement d'excitation doit être irradié vers le tissu,
   **caractérisé en ce que** ledit dispositif de commande (34) est configuré pour :

   déterminer une mesure de réponse/de contact (A), ladite mesure de réponse/de contact (A) indiquant un ou les deux d'un degré selon lequel ledit corps de mesure (16) présente une réponse physique lors de l'irradiation du rayonnement d'excitation vers le tissu afin qu'il soit absorbé dans celui-ci et d'un degré selon lequel ledit contact permet aux ondes de chaleur et/ou de pression générées par l'absorption du rayonnement d'excitation dans le tissu d'être transférées vers ledit corps de mesure (16) ; et
   contrôler ledit moyen (118, 502) afin de déplacer la surface de contact (14) et l'axe de propagation (112) du rayonnement d'excitation de manière transversale l'un par l'autre à l'autre sur la base de ladite mesure de réponse/de contact (A).

2. Dispositif (100, 200, 500) selon la revendication 1, dans lequel ledit analyte est du glucose, dans lequel, en particulier, ledit tissu est la peau du sujet et ledit analyte est le glucose présent dans un fluide interstitiel de celle-ci.

3. Dispositif (100, 200, 500) selon la revendication 1 ou 2, dans lequel le dispositif de commande (34) est configuré pour déterminer la mesure de réponse/de contact (A) sur la base d'un signal de réponse généré par ledit dispositif de détection (28, 30, 504), dans lequel, en particulier, le dispositif de commande (34) est configuré pour déterminer la mesure de réponse/de contact (A) en :

   contrôlant la source de rayonnement d'excitation (26) afin d'irradier le rayonnement d'excitation vers le tissu afin qu'il soit absorbé dans celui-ci à une ou plusieurs longueurs d'onde auxquelles le degré d'absorption dudit rayonnement d'excitation est sensiblement indépendant d'une concentration de l'analyte dans le tissu ; et en contrôlant le dispositif de détection (28, 30, 504) afin de détecter ladite réponse physique et de générer un signal de réponse qui indique le degré d'absorption dudit rayonnement d'excitation.

4. Dispositif (100, 200, 500) selon l'une quelconque des revendications 1 à 3, comprenant en outre un capteur auxiliaire (206) configuré pour générer un signal de capteur qui indique le degré selon lequel ledit contact permet aux ondes de chaleur et/ou de pression générées par l'absorption du rayonnement d'excitation dans le tissu d'être transférées vers ledit corps de mesure (16), dans lequel le dispositif de commande (34) est configuré pour déterminer la mesure de réponse/de contact (A) sur la base du signal de capteur généré par le capteur auxiliaire (206).

5. Dispositif (100, 200) selon l'une quelconque des revendications précédentes, dans lequel ledit moyen (118) configuré pour déplacer la surface de contact (14) et l'axe de propagation (112) du rayonnement d'excitation de manière transversale l'un par rapport à l'autre est ou comprend un moyen actionné (118) configuré pour déplacer la surface de contact (14) de manière transversale par rapport à l'axe de propagation (112) du rayonnement d'excitation irradié vers le tissu pendant que la surface de contact (14) est en contact avec la surface de peau (108) du sujet, dans lequel, en particulier, ledit moyen actionné (118) comprend :

un ou plusieurs actionneurs, ledit ou lesdits actionneurs comprenant un ou plusieurs d'un élément piézoélectrique, d'un actionneur électrique, d'un actionneur magnétique et d'une pompe à fluide ; et/ou
un ou plusieurs éléments déformables configurés pour être déformés, déployés et/ou rétractés, ledit ou lesdits éléments déformables comprenant un ou plusieurs d'un corps gonflable et/ou d'un élément déployable de manière pneumatique et/ou hydraulique.

6. Dispositif (100, 200) selon la revendication 5, dans lequel ledit moyen actionné (118) est configuré pour déplacer ladite surface de contact (14) en déplaçant le corps de mesure entier (16) ou un composant de celui-ci.

7. Dispositif (100, 200) selon la revendication 5 ou 6, dans lequel ledit dispositif de détection (28, 30) comprend une source de lumière destinée à générer un faisceau lumineux de détection (22) qui se déplace à travers au moins une partie dudit corps de mesure (16) ou d'un composant inclus dans celui-ci et ledit moyen actionné (118) est configuré pour déplacer ladite surface de contact (14) dans une direction qui est perpendiculaire ou sensiblement perpendiculaire à la fois à l'axe de propagation (112) dudit rayonnement d'excitation irradié vers le tissu et à un axe de propagation (202) dudit faisceau lumineux de détection (22), dans lequel, de préférence, ledit corps de mesure (16) comprend un prisme, en particulier un prisme trapézoïdal, ayant une première facette d'entrée (204A) qui est perpendiculaire ou sensiblement perpendiculaire à l'axe de propagation (112) dudit rayonnement d'excitation et une deuxième facette d'entrée (204B) qui est perpendiculaire ou sensiblement perpendiculaire à l'axe de propagation (202) dudit faisceau lumineux de détection (22), dans lequel ledit moyen actionné (118) est configuré pour déplacer ledit prisme avec ladite surface de contact (14) dans une direction parallèle ou sensiblement parallèle à la première et à la deuxième facettes d'entrée (204A, 204B).

8. Dispositif (100, 200) selon l'une quelconque des revendications 5 à 7, dans lequel ledit moyen actionné (118) est en outre configuré pour déplacer ladite surface de contact (14) longitudinalement par rapport à l'axe de propagation (112) dudit rayonnement d'excitation de façon à augmenter temporairement une pression de contact entre le corps de mesure (16) et la surface de peau (108) du sujet.

9. Procédé (300, 400) de détection d'un analyte dans un tissu d'un sujet humain ou animal à l'aide d'un dispositif (100, 200, 500) selon l'une quelconque des revendications précédentes, dans lequel la surface de contact (14) du corps de mesure (16) est en contact avec une surface de peau (108) du sujet et le procédé (300, 400) comprend :

la détermination d'une mesure de réponse/de contact (A), ladite mesure de réponse/de contact (A) indiquant un ou les deux d'un degré selon lequel ledit corps de mesure (16) présente une réponse physique lors de l'irradiation du rayonnement d'excitation vers le tissu afin qu'il soit absorbé dans celui-ci et d'un degré selon lequel ledit contact permet aux ondes de chaleur et/ou de pression générées par l'absorption du rayonnement d'excitation dans le tissu d'être transférées vers ledit corps de mesure (16) ;
le déplacement de la surface de peau (108) du sujet et de l'axe de propagation (112) du rayonnement d'excitation de manière transversale l'un par rapport à l'autre en déplaçant un ou les deux de la surface de contact (14) et de l'axe de propagation (112) du rayonnement d'excitation à l'aide dudit moyen (118, 502) avec la surface de contact (14) qui est en contact avec la surface de peau (108) du sujet afin de régler une position d'irradiation (114) sur la surface de peau (108) au niveau de laquelle ledit rayonnement d'excitation doit être irradié vers le tissu, dans lequel la surface de peau (108) du sujet et l'axe de propagation (112) du rayonnement d'excitation sont déplacés de manière transversale l'un par rapport à l'autre sur la base de ladite mesure de réponse/de contact (A) ; et
l'exécution d'une mesure d'analyte en irradiant le rayonnement d'excitation au niveau de la position d'irradiation réglée (114) vers le tissu afin qu'il soit absorbé par l'analyte contenu dans celui-ci à l'aide de la source de rayonnement d'excitation (26), en détectant la réponse physique du corps de mesure (16) ou d'un composant inclus dans celui-ci avec le dispositif de détection (28, 30, 504) et en générant un signal de réponse qui indique le degré d'absorption dudit rayonnement d'excitation à l'aide du dispositif de détection (28, 30, 504).

10. Procédé (300, 400) selon la revendication 9, dans lequel :

le procédé (300, 400) comprend en outre la comparaison de la mesure de réponse/de contact (A) avec un seuil de réponse/de contact, dans lequel la surface de peau (108) du sujet et l'axe de propagation (112) du rayonnement d'excitation sont déplacés de manière transversale l'un par rapport à l'autre en réponse à la détermination du fait que la mesure de réponse/de contact (A) ne satisfait pas le seuil de réponse/de contact ; et/ou

le procédé (300, 400) comprend en outre l'optimisation de la mesure de réponse/de contact (A) en déplaçant la surface de peau (108) du sujet et l'axe de propagation (112) du rayonnement d'excitation de manière transversale l'un par rapport à l'autre ; et/ou

la mesure de réponse/de contact (A) est déterminée de manière répétée et le procédé (300, 400) comprend en outre la réalisation d'une commande à asservissement active sur la base de ladite mesure de réponse/de contact (A) afin de régler ladite position d'irradiation (114) en déplaçant la surface de peau (108) du sujet et l'axe de propagation (112) du rayonnement d'excitation de manière transversale l'un par rapport à l'autre à l'aide dudit moyen (118, 502).

11. Procédé (300, 400) selon la revendication 9 ou 10, dans lequel la détermination de la mesure de réponse/de contact (A) comprend :

le contrôle de la source de rayonnement d'excitation (26) afin d'irradier le rayonnement d'excitation au niveau d'une position d'irradiation actuelle (114) vers le tissu afin qu'il soit absorbé dans celui-ci, en particulier à une ou plusieurs longueurs d'onde auxquelles le degré d'absorption dudit rayonnement d'excitation est sensiblement indépendant d'une concentration de l'analyte dans le tissu ;

le contrôle du dispositif de détection (28, 30, 504) afin de détecter ladite réponse physique et de générer un signal de réponse qui indique le degré d'absorption dudit rayonnement d'excitation ; et

la détermination de la mesure de réponse/de contact (A) sur la base dudit signal de réponse.

12. Procédé (300, 400) selon l'une quelconque des revendications 9 à 11, comprenant en outre la réalisation d'une autre mesure d'analyte au niveau d'une position d'irradiation initiale (114) avant de déplacer la surface de peau (108) du sujet et l'axe de propagation (112) du rayonnement d'excitation de manière transversale l'un par rapport à l'autre, dans lequel, en particulier, le procédé (300, 400) comprend la réalisation de manière répétée de mesures d'analyte et le déplacement de la surface de peau (108) du sujet et de l'axe de propagation (112) du rayonnement d'excitation de manière transversale l'un par rapport à l'autre entre lesdites mesures d'analyte afin d'échantillonner ou de scanner une pluralité de positions d'irradiation (114).

13. Dispositif (100, 200, 500) selon l'une quelconque des revendications 1 à 8, dans lequel le dispositif de commande (34) est configuré pour exécuter un procédé (300, 400) selon l'une quelconque des revendications 9 à 12.

**Fig. 1**

IR spectra of glucose at different concentrations

**Fig. 2**

**Fig. 3**

Clarke's Error Grid Analysis-raw Amplitude

1558 data points

20 subjects
of these
17 healthy,
2 type 1
diabetics,
1 type 2
diabetic

zone A: 95.4%
zone B: 4.0%
zone C: 0%
zone D: 0.6%
zone E: 0%

**Fig. 4**

**Fig. 5**

**Fig. 6**

Fig. 7

Fig. 8a

Fig. 8b

Fig. 9a

Fig. 9b

300

302 — determine
response/contact
measure

current position
on skin suitable for
measurement?

304

no → adjust position by
moving contact
surface and/or
propagation axis

306

yes

308 — perform
analyte measurement

Fig. 10

400

```
      ┌─────────────────────────┐
402 ──┤   perform first          │
      │   analyte measurement    │
      └─────────────────────────┘
                  │
                  ▼
      ┌─────────────────────────┐
404 ──┤ adjust position by moving│◄ ─ ─ ┐
      │ contact surface and/or   │      │
      │ propagation axis         │      │
      └─────────────────────────┘      │
                  │                     │
                  ▼                     │
      ┌─────────────────────────┐      │
406 ──┤   perform second         │ ─ ─ ─┘
      │   analyte measurement    │
      └─────────────────────────┘
                  │
                  ▼
      ┌─────────────────────────┐
408 ──┤ discard and/or average   │
      │ analyte measurements     │
      └─────────────────────────┘
```

Fig. 11

Fig. 12

**EP 4 505 940 B1**

**REFERENCES CITED IN THE DESCRIPTION**

**Patent documents cited in the description**

- WO 2015193310 A1
- WO 2017097824 A1
- WO 2020094265 A1
- WO 2019110597 A2
- US 2003010898 A1
- US 2023181064 A1
- WO 2023002024 A1
- US 2023190145 A1
- CN 208808486 U
- US 2008262324 A1
- WO 2021233559 A1
- WO 201709782 A1